Europäisches Patentamt

European Patent Office

Office européen des brevets

(11) Publication number: **0 308 936 B1**

## EUROPEAN PATENT SPECIFICATION

(45) Date of publication of patent specification: **06.07.94**

(51) Int. Cl.⁵: **A61K 39/395**, A61K 35/14,
A61K 37/66, //(A61K39/395,
35:14),(A61K37/66,39:395),
(A61K39/395,37:02)

(21) Application number: **88115607.9**

(22) Date of filing: **22.09.88**

The file contains technical information submitted
after the application was filed and not included in
this specification

(54) **Antibody heteroconjugates for the killing of HIV-infected cells.**

(30) Priority: **23.09.87 US 100157**

(43) Date of publication of application:
**29.03.89 Bulletin 89/13**

(45) Publication of the grant of the patent:
**06.07.94 Bulletin 94/27**

(84) Designated Contracting States:
**AT BE CH DE ES FR GB GR IT LI LU NL SE**

(56) References cited:
**EP-A- 0 180 171**
**EP-A- 0 255 249**
**WO-A-83/03679**
**WO-A-88/03565**
**US-A- 4 676 980**

**BIOLOGICAL ABSTRACTS, vol. 84, no. 2,
1987, Philadelphia, PA (US); L.H. GOSTING et
al., no. 21468&NUM;**

(73) Proprietor: **Bristol-Myers Squibb Company
345 Park Avenue
New York, N.Y. 10154(US)**

(72) Inventor: **Zarling, Joyce M.
1400 25th Avenue East
Seattle, WA 98112(US)**
Inventor: **Ledbetter, Jeffrey A.
30 Northwest 113th Place
Seattle, WA 98117(US)**

(74) Representative: **Kinzebach, Werner, Dr.
Patentanwälte
Reitstötter, Kinzebach und Partner
Postfach 86 06 49
D-81633 München (DE)**

THE JOURNAL OF IMMUNOLOGY, vol. 139, no. 2, 15 July 1987, American Association of Immunologists, US; G. JUNG et al., pp. 639-644&NUM;

BIOLOGICAL ABSTRACTS, vol. 83, no. 11, 1987, Philadelphia, PA (US); A. LANZAVEC-CHIA et al., no. 108039&NUM;

BIOTECHNOLOGY, vol. 5, September 1987; A. KLAUSNER, pp. 867-868&NUM;

THE JOURNAL OF IMMUNOLOGY, vol. 140, no. 8, 15 April 1988, American Association of Immunologists, US; J.M. ZARLING et al., pp. 2609-2613&NUM;

**Description**

TECHNICAL FIELD OF THE INVENTION

The present invention relates to novel antibody heteroconjugates and their use in methods for killing cells infected with Human Immunodeficiency Virus (HIV) in the treatment of HIV infections. More particularly, the invention relates to the construction of antibody heteroconjugates comprising an antibody specific for a particular peripheral blood effector cell cross-linked to an antibody specific for an HIV antigen present on the surface of HIV-infected cells. Such antibody heteroconjugates physically bridge the effector cell to the target cell to be killed and may activate the lytic mechanism of the effector cell in the killing of the HIV-infected target cell. The antibody heteroconjugates and methods of this invention provide a novel approach to the treatment of HIV-infected individuals by amplifying endogenous HIV-specific effector mechanisms and may also be of prophylactic value, before the development of HIV immune responses, in individuals newly infected or accidentally exposed to the HIV virus.

BACKGROUND OF THE INVENTION

The infectious agents responsible for acquired immunodeficiency syndrome (AIDS) and its prodromal phases, AIDS-related complex (ARC) and lymphadenopathy syndrome (LAS), are novel lymphotropic retroviruses recently termed Human Immunodeficiency Virus (HIV 1 and 2). Isolates of these viruses include LAV-1, LAV-2, HTLV-III, and ARV.

The general structure of HIV is that of a ribonucleoprotein core surrounded by a lipid-containing envelope which the virus acquires during the course of budding from the membrane of the infected host cell. Embedded within the envelope and projecting outward are the viral-encoded glycoproteins. For example, the envelope glycoproteins of HIV-1 are initially synthesized in the infected cell as a precursor molecule of 150,000-160,000 daltons (gp150 or gp160), which is then processed in the cell into an N-terminal fragment of 110,000-120,000 daltons (known in the art as gp110 or gp120) to generate the external glycoprotein, and a C-terminal fragment of 41,000-46,000 daltons (gp41), which represents the transmembrane envelope glycoprotein. The internal viral proteins of HIV include the "gag" and "pol" proteins.

As the spread of HIV reaches pandemic proportions, the treatment of infected individuals and prevention of the virus' transmission to uninfected individuals at risk of exposure is of paramount concern. A variety of therapeutic strategies have targeted different stages in the life cycle of the virus and are outlined in Mitsuya and Broder, Nature, 325, p. 773 (1987). One approach involves the use of antibodies specific for antigens on the HIV glycoproteins. These antibodies may inhibit viral replication, either by interfering with viral entry into host cells or by some other mechanism. Once the viral proteins or the antigenic determinants on those proteins that are susceptible to antibody intervention are identified, antibody titers sufficient to neutralize the infectivity of the virus could be engendered by vaccination or, alternatively, by the passive administration of immunoglobulins or monoclonal antibodies of the desired antigenic specificity.

The gp110 glycoprotein of HIV-1 has been the object of much investigation as a potential target for interfering with the virus' infectivity. Sera from HIV-infected individuals have been shown to neutralize HIV in vitro and antibodies that bind to purified gp110 are present in the sera [see M. Robert-Guroff et al., Nature, 316, pp. 72-74 (1985); R.A. Weiss et al., Nature, 316, pp. 69-72 (1985); and Mathews et al., Proc. Natl. Acad. Sci. U.S.A., 83, p. 9709 (1986)]. Purified and recombinant gp110 have stimulated the production of neutralizing serum antibodies when used to immunize animals [see Robey et al., Proc. Natl. Acad. Sci. U.S.A., 83, p. 7023 (1986) and Lasky et al., Science, 233, p. 209 (1986)]. Immunization of a human with a recombinant vaccinia virus that expresses HIV gp110 and gp41 induced HIV-neutralizing antibodies [see Zagury et al., Nature, 326, p. 249 (1986)]. Binding of the gp110 molecule to the CD4 (T4) receptor has also been shown and monoclonal antibodies which recognize certain epitopes of the CD4 receptor have been shown to block HIV binding, syncytia formation and infectivity [see McDougal et al., Science, 231, p. 382 (1986)]. Putney et al., Science, 234, p. 1392 (1986) elicited neutralizing serum antibodies in animals after immunizing with a recombinant fusion protein containing the carboxyl-terminal half of the gp110 molecule and further demonstrated that glycosylation of the envelope protein is unnecessary for a neutralizing antibody response. Furthermore, monoclonal antibodies to HIV glycoproteins such as gp110 and gp41 have been produced [see, e.g., L.H. Gosting et al., J. Clin. Microbiol., 25, (No. 5), pp. 845-848 (1987)].

HIV may spread in an infected individual in two ways: as cell-free virus present in body fluids or by fusion of infected cells with uninfected cells to form syncytia. Although neutralizing antibody to HIV may reduce the spread of cell-free virus, a different approach is required to kill HIV-infected cells.

Ordinarily, a healthy immune system enables an individual to recover from infections caused by a variety of viruses. The immune mechanisms which may play a role in prevention or recovery from viral infections include the following: First, neutralizing antibodies may prevent the spread of cell-free virus. Second, antibody dependent cell-mediated cytotoxicity (ADCC), which involves coating of virus-infected cells with antibody, enables effector cells within the peripheral blood leukocyte population to lyse the infected cells. Third, complement-dependent antibody cytotoxicity may contribute to the lysis of virus-infected cells. Fourth, T cell-mediated immunity may also be involved in the killing of virus-infected cells [see Introduction To Immunology (2nd ed.), J.W. Kimball (ed.), MacMillan Publishing Co. (1986) for a general review of the cells and functions of the human immune system].

However, the HIV virus itself causes immunodeficiencies. Thus, a large proportion of HIV-infected humans go on to develop AIDS, indicating that the immune responses to HIV in those individuals are inadequate to prevent the development of this fatal disease. Therefore, there is a great need, particularly in the treatment of HIV infections, for a new approach that will enable or augment effector cell mechanisms for the killing of HIV-infected cells.

The use of antibody heteroconjugates (also known in the art as heteroaggregates or heteroantibodies) for the killing of tumor cells is known. See, e.g., EP-A-0 180 171, United States Patent 4,676,980, issued to D.M. Segal et al.; G. Jung et al., Proc. Natl. Acad. Sci. U.S.A., 83, pp. 4479-4483 (1983); P. Perez et al., J. Immunol., 137, No. 7, pp. 2069-2072 (1986); and J.A. Titus et al., J. Immunol., 138, No. 11, pp. 4018-4022 (1987). For example, Perez et al., supra, disclose the cross-linking of anti-T3, a monoclonal antibody to the T receptor on T lymphocytes, to various anti-tumor monoclonal antibodies. The heteroaggregates so produced were shown to promote the lysis of human tumor lines and fresh tumor cells by cytotoxic T lymphocytes. See also, B. Karpovsky et al., J. Exp. Med., 160, pp. 1686-1701 (1984), which discloses heteroaggregates containing antibodies to the Fc receptor on ADCC effector cells cross-linked to antibodies to TNP-treated tumor cells for the lysis of those tumor cells.

However, no where in the art is there a suggestion or a teaching as to how to select and use monoclonal antibodies specific for HIV antigens cross-linked to effector cells such as cytotoxic T lymphocytes or large granular lymphocytes for the killing of HIV-infected cells in the treatment of HIV infections. Furthermore, because of the immune deficiencies caused by the HIV virus, one would not be led by previous studies to use the heteroconjugate approach in AIDS patients.

SUMMARY OF THE INVENTION

The present invention relates to the use of novel antibody heteroconjugates for the treatment of HIV-infected individuals. The heteroconjugates of the invention are comprised of an antibody specific for an HIV antigen that is expressed on HIV-infected cells cross-linked to an antibody specific for an effector cell of the peripheral blood capable of killing an HIV-infected target cell. According to the method of this invention, the anti-HIV antibody of the heteroconjugate binds to an HIV-infected cell, i.e., the target cell to be killed, whereas the anti-effector antibody of the heteroconjugate binds to an effector cell such as those found within the peripheral blood lymphocyte (PBL) population, e.g., cytotoxic T lymphocytes (also referred to in the art as T cells) or large granular lymphocytes (LGLs). Thus, the antibody components of the heteroconjugate bridge the effector and target cells and promote killing of the target cell by the cytotoxic effector cell.

According to preferred embodiments of this invention, monoclonal antibodies to either gp110 or gp41 of HIV-1 have been cross-linked to either a monoclonal antibody specific for the CD3/T cell receptor complex found on T lymphocytes or a monoclonal antibody to the Fc receptor found on certain leukocytes to form heteroconjugates that target the respective effector cells (e.g., T lymphocytes or LGLs) from HIV seropositive or seronegative humans to kill HIV-infected cells.

The heteroconjugates of this invention may be used in anti-HIV compositions, such as those comprising a pharmaceutically effective amount of at least one heteroconjugate of the invention. The present invention also encompasses combinations for use in methods for treating HIV-infected individuals comprising the step of treating the individual in a pharmaceutically acceptable manner with a pharmaceutically effective amount of the compositions of this invention. In addition to in vivo treatment of HIV-infected individuals with the heteroconjugates, a method for treating HIV-infected individuals involves the in vitro activation of effector cells such as peripheral blood lymphocytes and administration of the activated effector cells and the heteroconjugates to the HIV-infected patient.

The heteroconjugates, pharmaceutical compositions and combinations of this invention are useful for killing HIV-infected cells in individuals suffering from HIV infections and may be particularly useful if the heteroconjugates contain one or more antibodies that neutralize HIV infectivity or if the heteroconjugates are administered together with HIV-neutralizing antibodies. In addition, these heteroconjugates may be of prophylactic value in the treatment of individuals newly or accidentally infected with HIV.

## BRIEF DESCRIPTION OF THE DRAWINGS

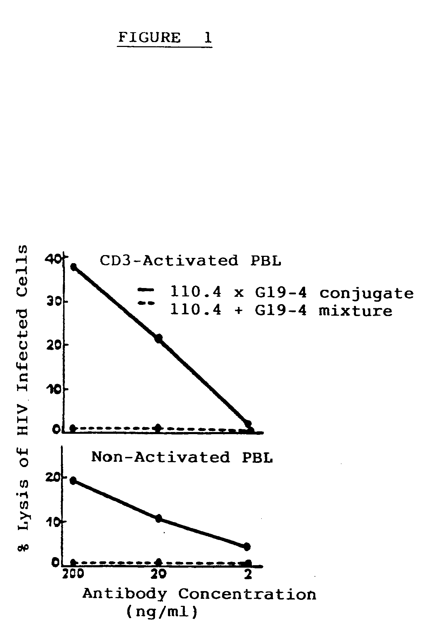

Figure 1 depicts a comparative graphical presentation of the % lysis of HIV-infected cells by anti-CD3-pretreated or untreated PBL from seronegative donors vs. antibody concentration of either the 110.4 x G19-4 heteroconjugate of one embodiment of this invention or a control mixture of the individual antibodies that make up the heteroconjugate.

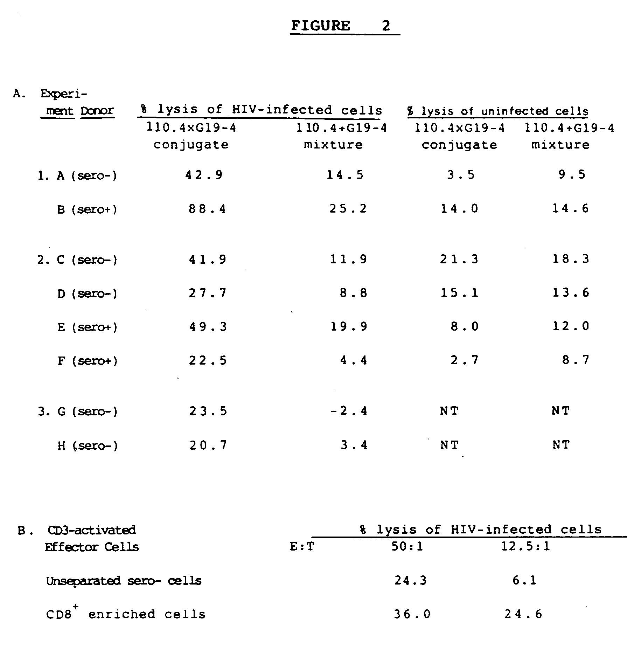

Figure 2A depicts in table form the % lysis of HIV-infected and uninfected cells by anti-CD3-pretreated PBL from seropositive and seronegative donors in the presence of either the 110.4 x G19-4 heteroconjugate of one embodiment of the invention or a control mixture of the individual antibodies that make up the heteroconjugate (NT = not tested).

Figure 2B depicts in table form the % of HIV-infected cells by anti-CD3-pretreated unseparated or CD8+-enriched PBL from seronegative donors in the presence of the 110.4 x G19-4 heteroconjugate of one embodiment of this invention.

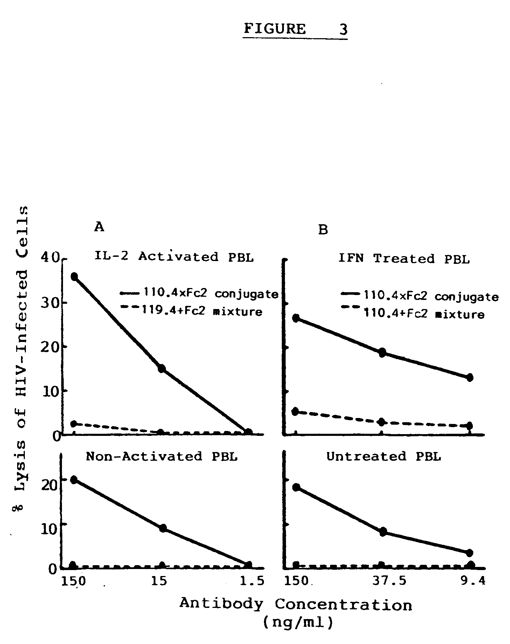

Figure 3 depicts a comparative graphical presentation of the % lysis of HIV-infected cells by untreated or (A) interleukin-2 (IL-2)-pretreated or (B) $\beta$-interferon ($\beta$-IFN)-pretreated PBL from seronegative donors vs. antibody concentration of either the 110.4 x Fc2 heteroconjugate of one embodiment of this invention or a control mixture of the individual antibodies that make up the heteroconjugate.

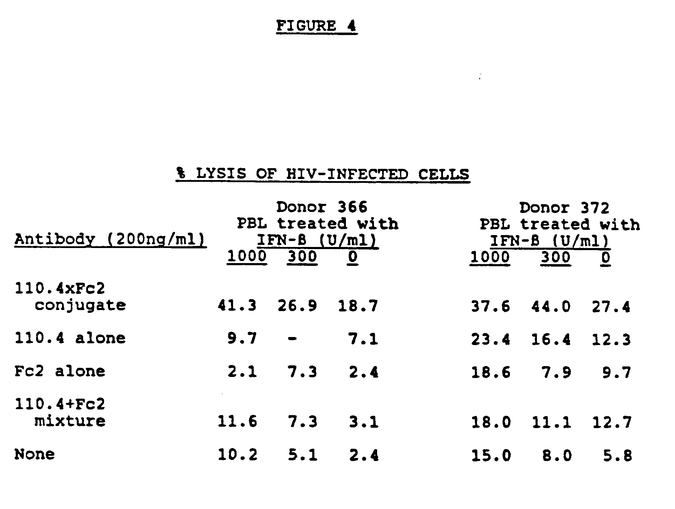

Figure 4 depicts in table form the % lysis of HIV-infected cells by PBL from seronegative donors, that were pretreated for 3 hours with varying concentrations of $\beta$-IFN, in the presence of the 110.4 x Fc2 heteroconjugate of one embodiment of the invention, a mixture of the individual antibodies that make up the heteroconjugate, each individual antibody of the conjugate alone or in the absence of any heteroconjugate or antibodies.

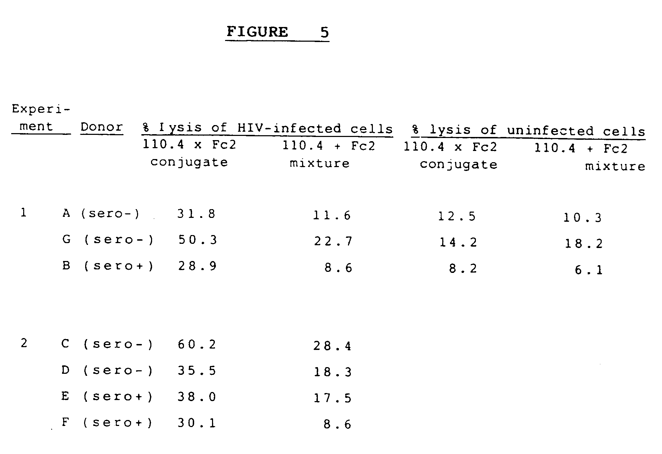

Figure 5 depicts in table form the % lysis of HIV-infected and uninfected cells by IL-2-pretreated seropositive and seronegative PBL in the presence of either the 110.4 x Fc2 heteroconjugate of one embodiment of this invention or a control mixture of the individual antibodies that make up the heteroconjugate.

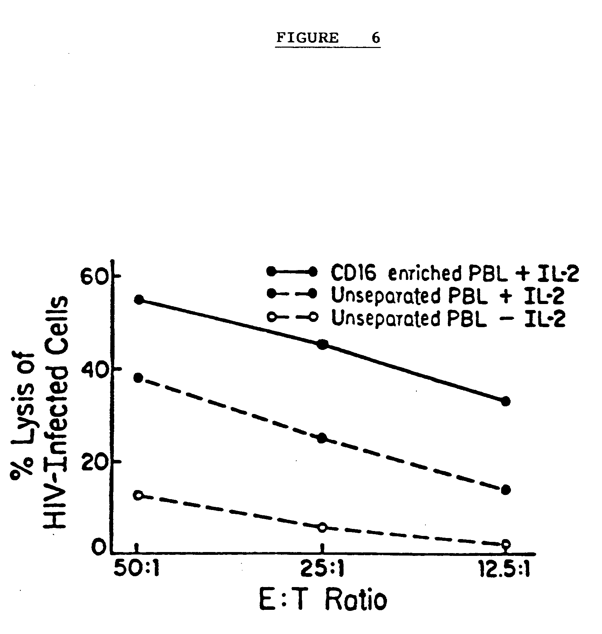

Figure 6 depicts a comparative graphical presentation of the % lysis of HIV-infected cells by CD16+-enriched seronegative PBL pretreated with IL-2, unseparated seronegative PBL pretreated with IL-2, and untreated unseparated seronegative PBL, in the presence of the 110.4 x Fc2 heteroconjugate over a range of effector:target (E:T) cell ratios.

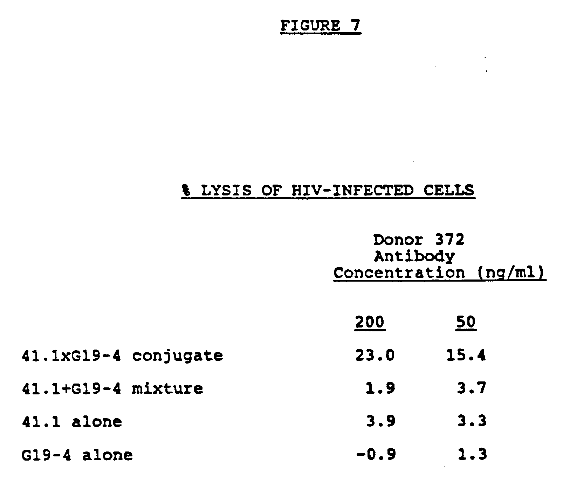

Figure 7 depicts in table form the % lysis of HIV-infected cells by PBL from seronegative donors in the presence of varying concentrations of either the 41.1 x G19-4 heteroconjugate of one embodiment of the invention, a mixture of the individual antibodies that make up the heteroconjugate or each individual antibody of the heteroconjugate alone.

## DETAILED DESCRIPTION OF THE INVENTION

In order that the invention herein described may be more fully understood, the following detailed description is set forth.

The present invention relates to novel antibody heteroconjugates and their use in methods for killing HIV-infected cells. More particularly, the invention relates to heteroconjugates comprised of at least two antibodies cross-linked to each other. One antibody is specific for and reactive with an HIV antigen expressed on HIV-infected cells. The other antibody is specific for and reactive with an antigen found on effector cells of the peripheral blood capable of killing an HIV-infected target cell. Such effector cells may include cytotoxic T cells, monocytes (or macrophages), granulocytes, and LGLs, which include cells with natural killer (NK) cell activity or ADCC activity. Since the HIV-specific antibody of the heteroconjugate binds to HIV-infected cells and the effector cell-specific antibody of the heteroconjugate binds to the cytotoxic effector cell, the heteroconjugate of this invention provides a means to bridge the two cells, bringing the cytotoxic effector cell in contact with the infected target cell and thus promoting lysis of the target cell.

Without being bound by theory, it is believed that the heteroconjugates described herein act not only to bridge cytotoxic effector cells to the HIV-infected target cells but also activate the lytic mechanisms of the effector cells [see, e.g., M.A. Liu et al., Proc. Natl. Acad. Sci. U.S.A., 82, pp. 8648-8652 (1985) and P. Perez et al., J. Exp. Med., 163, pp. 166-178 (1986)]. Thus, although an effector cell such as a T cell may have its own antigenic specificity, it can be retargeted by the interaction with the heteroconjugate of this invention to kill HIV-infected cells bound by the heteroconjugate. The heteroconjugate approach of this invention,

5

therefore, provides for an enhanced HIV-specific effector cell response in HIV-infected individuals by activating effector cells such as cytotoxic T cells or LGLs and then bringing them into close proximity, via the antibody heteroconjugate bridge, with the HIV-infected target cells to be killed. In addition, such heteroconjugates can render effector cells from individuals who have not yet developed anti-HIV immunity (e.g., newly infected individuals) cytotoxic because naive effector cells can be targeted by the heteroconjugates of this invention to kill HIV-infected cells.

The antibodies that comprise the heteroconjugates of this invention may be polyclonal or preferably, monoclonal. The term "antibody" as used in this application includes intact antibody molecules or Fab or F-(ab')$_2$ fragments. If monoclonal antibodies are used, the antibodies may be of mouse or human origin or chimeric antibodies. The antibodies that comprise the heteroconjugates of this invention can be covalently bound to each other by techniques well known in the art such as the use of the heterobifunctional cross-linking reagent SPDP (N-succinimidyl-3-(2-pyridyldithio)propionate) [see e.g., B. Karpovsky et al., supra]. Alternatively, the antibodies may be covalently bound to each other using GMBS (maleimido butryloxysuccinimide) as described by R.R. Hardy, in Methods Immunol., 4th Ed., D.M. Weir (ed.), pp. 31.1-31.12 (1986). Furthermore, an embodiment of the invention may involve heteroconjugates comprised of more than two antibodies. For example, a heteroconjugate of the invention may be comprised of two antibodies specific for the effector cell and one antibody specific for the target HIV-infected cell. Alternatively, the heteroconjugate may be comprised of two antibodies specific for the target cell and one antibody specific for the effector cell. These heteroconjugates are covalently bound to each other by techniques known in the art as cited above.

The HIV-specific antibody of the heteroconjugate may be any antibody that is specific for and reactive with an HIV antigen that is sufficiently exposed or expressed on the surface of HIV-infected cells. The antibody should, additionally, have an affinity for the HIV antigen on the cell surface such that the antibody heteroconjugate forms a stable bridge between the infected target cell and the effector cell. The antibody should preferably have an affinity association constant on the order of about $10^{-8}$ to about $10^{-12}$.

The effector-specific antibody of the heteroconjugate may be any antibody that is specific for and reactive with an effector cell of the peripheral blood capable of killing an HIV-infected cell. Preferably, the antibody is one that reacts with an antigen on the surface of the effector cell such that the lytic mechanism of the effector cell is activated. Such antibodies may include antibodies that react with epitopes on T lymphocytes such as CD3 [see S.C. Meuer et al., J. Exp. Med., 157, p. 705 (1983)], CD28 (also known in the art as Tp44) [see J.A. Ledbetter et al., J. Immunol., 137, pp. 3299-3305 (1986) and Leukocyte Typing III, A.J. McMichael (ed.), Oxford University Press, Oxford (in press)], and CD2 [see C.H. June et al., J. Clin. Invest., 77, p. 1224 (1986) and Leukocyte Typing, A. Bernard et al. (ed.s), Springer-Verlag, New York (1984)]. Alternatively, the effector-specific antibody component of the heteroconjugate may include antibodies that react with epitopes on the Fc receptors of certain effector cells such as LGLs, granulocytes or monocytes. Examples of such antibodies include antibodies specific for the CD3/T cell receptor complex on T lymphocytes such as the G19-4 antibody [see, e.g., J.A. Ledbetter et al., J. Immunol., 135, pp. 2331-2336 (1985)] and antibodies that react with the CD16 Fc receptor of LGLs such as the Fc2 antibody [see, e.g., J.A. Ledbetter et al., In Perspectives In Immunogenetics And Histocompatibility, Vol. 6, E. Heise (ed.), Lymphocyte Surface Antigens 1984, pp. 325-340, American Society For Histocompatibility And Immunogenetics, New York (1984)]. Both anti-CD3 and anti-CD16 monoclonal antibodies are commercially available (e.g., Leu 4 and Leu 11 antibodies, respectively, Becton Dickinson, Mountainview, CA).

According to one preferred embodiment of this invention, a monoclonal antibody to the HIV-1 glycoprotein gp110 (110.4) was cross-linked to a monoclonal antibody to the CD3 antigen found on the T cell receptor (G19-4). The heteroconjugate targeted T cells from PBL of seropositive and seronegative humans to kill HIV-infected cells.

According to our experimental protocol, PBL were incubated with radiolabeled chromium ($^{51}$Cr) HIV-infected target cells in the presence of a 110.4 x G19-4 heteroconjugate of this invention and lysis of the target cells determined by the release of the $^{51}$Cr label into the medium. We found that the PBL lysed the HIV-infected cells in the presence of the heteroconjugate whereas little or no lysis occurred in the presence of a mere mixture of the individual monoclonal antibodies that made up the conjugate.

In addition, we found that PBL preincubated with anti-CD3 and then exposed to the target cells in the presence of the 110.4 x G19-4 heteroconjugate were even more cytotoxic to the HIV-infected cells than untreated PBL. This result is in agreement with studies directed to the use of heteroconjugates against tumor cells which reported that pretreatment with antibody to the T3 antigen on T cells stimulates or augments the lytic mechanism of the T cell [see, e.g., G. Jung et al., supra].

Of great importance was the fact that PBL from asymptomatic HIV-infected, i.e., seropositive individuals, were also capable of lysing HIV-infected cells in the presence of the 110.4 x G19-4 heteroconjugate. Thus,

6

the heteroconjugates of this invention may provide a means for enabling or augmenting the ability of effector cells in the blood of individuals already infected with HIV to kill HIV-infected cells.

Furthermore, we found that anti-CD3-activated PBL enriched for CD8 + cells (CD8 being an antigenic marker for cytotoxic T cells) were more cytotoxic then unfractionated PBL. This observation suggests that CD8+ cytotoxic T cells within the PBL population are targeted by the 110.4 x G19-4 heteroconjugate for the lysis of the HIV-infected cells.

In a second preferred embodiment, a monoclonal antibody to gp110 (110.4) was cross-linked to Fc2, a monoclonal antibody to CD16, an antigen identified as the Fc receptor expressed on LGLs and granulocytes. LGLs are effector cells that mediate ADCC and natural killing [see, e.g., C. Ohlander et al., Scand. J. Immunol., 15, pp. 409-415 (1982)]. The heteroconjugate targeted LGLs from PBL of seropositive and seronegative individuals to kill HIV-infected cells.

Thus, PBL were incubated with $^{51}$Cr-labeled HIV-infected cells in the presence of a 110.4 x Fc2 heteroconjugate and lysis determined by release of $^{51}$Cr. The PBL lysed the HIV-infected cells in the presence of the heteroconjugates. Less lysis was observed in control experiments where the PBL were incubated with target cells in the presence of a mere mixture of the monoclonal antibodies that make up the 110.4 x Fc2 heteroconjugate. Furthermore, as with the preceding embodiment, PBL from HIV seropositive humans were also targeted to lyse HIV-infected cells in the presence of the 110.4 x Fc2 heteroconjugate.

To determine which cells within the PBL population were actually targeted by this heteroconjugate, PBL enriched for CD8+ T cells and PBL enriched for CD16+ cells were tested for their ability to lyse HIV-infected cells. PBL enriched for CD16+ cells, mainly LGLs, were more cytotoxic for the HIV-infected cells than unfractionated PBL. In fact, PBL enriched for CD8+ T cells were less cytotoxic than the unfractionated population. Thus, CD16+ LGLs present within the PBL population lysed the HIV-infected cells in the presence of the 110.4 x Fc2 heteroconjugate.

The present invention makes possible the treatment of HIV-infected individuals with pharmaceutical compositions comprising the heteroconjugates of this invention. This method of treatment may be carried out in vivo by the administration to an HIV-infected individual of a pharmaceutically effective amount of at least one antibody heteroconjugate of the invention. The administration of the heteroconjugate in conjunction with or after treatment with β-interferon (β-IFN), interleukin 2 (IL-2), other interferons such as α- or γ-interferon, interferon inducers or other immunomodulators may augment the effectiveness of the treatment. For example, our experiments have shown that pretreatment of PBL with β-IFN or IL-2 caused more lysis of HIV-infected cells in the presence of the heteroconjugates of this invention than the lysis observed with untreated PBL. It may also be desireable to treat HIV-infected individuals with the heteroconjugates of this invention wherein the heteroconjugates themselves are comprised of HIV-neutralizing antibodies (i.e., as the HIV-specific antibody component of the heteroconjugate) or with the heteroconjugates in conjunction with HIV-neutralizing antibodies to enhance the body's overall attack on the HIV virus.

Alternatively, the treatment of HIV-infected individuals may involve the steps of treating effector cells of the peripheral blood such as PBL with at least one antibody heteroconjugate of the invention in vitro and administering the effector cells and the heteroconjugate to the HIV-infected individual. This method of treatment may also involve the in vitro co-incubation or preincubation of the effector cells with β-IFN or IL-2, other interferons such as α- or γ-interferon, interferon inducers or other immunomodulators, and the administration of the activated effector cells with the heteroconjugates to an HIV-infected individual. Alternatively, the effector cells may be co-incubated or preincubated in vitro with an antibody specific for and reactive with the particular effector cell utilized; preferably, an antibody that stimulates the lytic mechanism of the effector cell, resulting in the cell's activation. For example, when using heteroconjugates comprising antibodies to cytotoxic T cells, treatment may include co-incubation or preincubation of the effector cells with an antibody specific for T cells because of the studies that indicate that such treatment may further stimulate the lytic mechanism of cytotoxic T cells. Finally, the effector cells can also be pretreated with a mitogen such as PHA or ConA before administration along with the heteroconjugate to the HIV-infected patient. Regardless of the method of treatment, it may be useful to use heteroconjugates comprising antibody fragments such as Fab or F(ab')$_2$ or chimeric antibodies.

The heteroconjugates of the invention can be administered using conventional modes of administration which include, but are not limited to, intravenous, oral, subcutaneous, intraperitoneal or intralymphatic. Intravenous administration is preferred.

The pharmaceutical compositions of the invention --comprising the heteroconjugates -- may be in a variety of dosage forms which include, but are not limited to, solid, semi-solid and liquid dosage forms such as tablets, pills, powders, liquid solutions or suspensions, suppositories, polymeric microcapsules or microvesicles, liposomes, and injectable or infusible solutions. The preferred form depends upon the mode of administration and the therapeutic application.

The heteroconjugate compositions may include conventional pharmaceutically acceptable carriers known in the art such as serum proteins such as human serum albumin, buffer substances such as phosphates, water or salts or electrolytes.

The most effective mode of administration and dosage regimen for the heteroconjugate compositions of this invention depends upon the severity and course of the disease, the patient's health and response to treatment and the judgement of the treating physician. Accordingly, the dosages of the heteroconjugates and any accompanying compounds such as $\beta$-IFN or IL-2 should be titrated to the individual patient.

Nevertheless, an effective dose of heteroconjugate of this invention may be in the range of from about 1 to about 100 mg/m$^2$. For in vitro treatment of effector cells, a dose of from about 200 $\mu$g - 2 mg of heteroconjugate/10$^9$ cells administered may be used. An effective dose of $\beta$-IFN, $\alpha$-IFN, or $\gamma$-IFN may be in the range of about 3X10$^6$ U/patient to about 360x10$^6$ U/patient with an optimum dose of 1x10$^7$ U/patient. Intravenous administration is preferred when using $\beta$-IFN, whereas subcutaneous administration is preferred when using $\alpha$- or $\gamma$-IFN. And, an effective dose of IL-2 may be in the range of about 1000 to about 100,000 U/kg body weight. Using a constant infusion, an effective dose may be from about 1-7 x 10$^6$ U per square meter of body surface per day [see W.H. West et al., New Eng. J. Med., 316 (No. 15), pp. 898-905 (1987)]. Finally, an effective dose of HIV-neutralizing antibody may be in the range of about 1 to about 100 mg/m$^2$.

In order that the invention described herein may be more fully understood, the following examples are set forth. It should be understood that these examples are for illustrative purposes only and are not to be construed as limiting the scope of this invention in any manner.

EXAMPLE 1

The following example demonstrates the targeting of PBL from HIV seropositive and seronegative individuals by the heteroconjugates of this invention for the lysis of HIV-infected cells.

The monoclonal antibodies cross-linked to form a heteroconjugate according to this invention were an HIV-specific antibody, 110.4, and a T cell-specific, anti-CD3 antibody, G19-4. Antibody 110.4 is of subclass IgG$_1$, reacts with the gp110 glycoprotein of LAV within the region coded for by nucleotides 6598-7178 of LAV [see L.H. Gosting et al., supra], and neutralizes the infectivity of HIV. G19-4 is of subclass IgG$_1$ and is specific for the CD3 antigen on the T cell receptor of T lymphocytes.

Monoclonal antibody, 110.4, was prepared as follows: LAV-1 virus purified from infected CEM cells (A.T.T.C. No. CRL8904) [see F. Barre-Sinoussi et al., Science, 220, pp. 868-871 (1983)] was disrupted in 50 mM Tris, pH 7.4, 0.15 M NaCl, 1.0% Aprotinin, 2.0% Nonidet P-40$^{(R)}$(NP-40) (octylphenoxypolyethoxyethanol). The extract was clarified twice by centrifugation and adjusted to 0.5% NP-40 with the addition of three volumes of disruption buffer without NP-40. Lentil lectin Sepharose® (Pharmacia, Piscataway, N.J.) was prewashed in disruption buffer without NP-40 and then equilibrated in adsorption buffer (50 mM Tris, pH 7.4, 0.15 M NaCl, 1.0% Aprotinin, 0.5% NP-40). Clarified viral extract was adsorbed with lentil lectin Sepharose® for 42 hours at 4°C. Unadsorbed material was removed by washing with excess adsorption buffer. Elution of adsorbed material was carried out with 0.2 M alpha methyl mannoside in adsorption buffer. The eluent was dialyzed against PBS to remove the sugar and the material was readsorbed to the lentil lectin Sepharose®.

The glycoprotein-lentil lectin Sepharose® complex was used to immunize BALB/c mice by three intraperitoneal injections without adjuvant given 2-3 weeks apart. Spleens were removed from immunized mice that demonstrated circulating antibody to glycoproteins of HIV by immunoblot, radioimmunoprecipitation and/or ELISA.

The procedures used for the generation of hybridoma cell lines were generally those of Kohler and Milstein, Nature, 256, p. 495 (1975) with the modifications of Goldstein et al., Infect. Immun., 38, p. 273 (1982). Splenic B lymphocytes from the immunized mice were fused with NS-1 myeloma cells using 40% (w/v) polyethylene glycol. Following fusion, the cell mixture was resuspended in HAT medium (RPMI-1640 medium supplemented with 15% fetal calf serum, 1x10$^{-4}$ M hypoxanthine, 4x10$^7$ M aminopterin and 1.6x10$^{-5}$ M thymidine) to select for the growth of hybrid cells, and then dispensed into 96-well microculture trays at a concentration of 1-3x10$^6$ cells/ml and incubated at 37°C in a humidified atmosphere containing 6% CO$_2$. Cultures were fed by replacement of one-half the supernatant with fresh HAT medium. The wells were observed using an inverted microscope for signs of cell proliferation and when the cells were of sufficient density, the supernatants were tested for anti-LAV antibody.

Wells containing hybrid cells producing antibody to LAV were identified by ELISAs measuring the binding to either purified whole disrupted virus or biologically-expressed fusion proteins. ELISA assays using disrupted virus were carried out on LAV EIA plates (Genetic Systems, Seattle, Washington). Plates were incubated with cell culture fluids at 37°C for 45 minutes and then washed three times with 0.05% Tween®20 in phosphate buffered saline (PBS-Tween®).

Peroxidase-goat anti-mouse IgG (1:2,000 dilution in PBS-Tween®; Zymed Laboratories, Inc., South San Francisco, California) was added (100 ul per well) and the plates were incubated for 45 minutes at 37°C and washed as above. Substrate (0.025 M citric acid, 0.05 M dibasic sodium phosphate, pH 5.0, containing 14 mg of o-phenylenediamine and 10 ul of 30% hydrogen peroxide per 50 ml) was added and the plates were incubated for 30 minutes at room temperature in the dark. The reaction was stopped with 3N sulfuric acid, and colorimetric reactions were quantitated with an automated microplate reader. Wells that gave positive results were subcloned by limiting dilution, retested for specificity, and then expanded.

The monoclonal antibodies secreted by the resulting hybrid cell lines were further characterized as to specificity and reactivity by immunoblotting, immunoprecipitation and ELISA using disrupted LAV virus, recombinant LAV fusion proteins and synthetic LAV peptides. All antibodies were determined to be of the $IgG_1$ isotype. The hybridoma that produced the 110.4 antibody utilized in this embodiment was deposited with the American Type Culture Collection under A.T.C.C. No. HB9405 in connection with commonly-owned GB 2 196 634 B. In addition, the production of monoclonal antibodies to the gp110 and gp41 glycoproteins of HIV-1 has been described by L.H. Gosting et al., J. Clin. Microbiol., 25 (No. 5), pp. 845-848 (1987). The anti-CD3 monoclonal antibody, G19-4, was prepared as described by J.A. Ledbetter and E. Clark, Human Immunology, 15, pp. 30-43 (1986). In addition, monoclonal antibodies to the CD3 antigen are also commercially available [see, e.g., P. Perez et al., 1986, supra]. The hybridoma that produces the particular anti-CD3 monoclonal antibody utilized in this embodiment of the invention, i.e., G19-4, was deposited with the American Type Culture Collection prior to the filing of this application.

The 110.4 and G19-4 monoclonal antibodies were cross-linked according to the method of B. Karpovsky et al., supra, using SPDP, and separated from free antibody by Sephacryl® S300 size exclusion chromatography. Fractions containing high molecular weight conjugates of >300 Kd were tested in immunofluorescence assays [see, e.g., J.A. Ledbetter et al., J. Exp. Med., 152, pp. 280-295 (1980)] for reactivity with a) CD3 on viable human PBL and b) acetone-fixed CEM cells that had been infected with LAV-1. Fractions with the highest binding activity to both the CD3 and the HIV antigens were then used in $^{51}$Cr-release cytotoxicity assays to test the ability of PBL from HIV seropositive or seronegative individuals to lyse HIV-infected CEM cells in the presence of the 110.4 x G19-4 heteroconjugate.

The cytotoxicity assay was performed as follows: CEM cells were infected with the LAV-1 isolate for 48 hours until virtually 100% of the cells expressed gp110 as determined by indirect immunofluorescence using monoclonal antibody 110.4 followed by treatment with fluorescein isothiocyanate-labeled goat anti-mouse immunoglobulin G F(ab')$_2$ (Zymed). The infected cells were then labeled for 1 hour with $^{51}$Cr (Na$_2$CrO$_4$, New England Nuclear, Boston, MA) and used as target cells in the assay.

The effector cells were ficoll-hypaque purified PBL from HIV seronegative or seropositive individuals. The PBL were cultured with or without monomeric anti-CD3 (G19-4) on solid phase for 3 days and then in anti-CD3-free medium for 24 hours. The untreated and treated PBL were then incubated for 4 hours at 37°C with 3x10$^3$ $^{51}$Cr-labeled target CEM cells in 96-well microtiter plates at an effector:target cell (E:T) ratio of 50:1 with varying concentrations of the 110.4 x G19-4 heteroconjugate or mere mixtures of the individual 110.4 and G19-4 antibodies as a control. Supernatants were harvested and counted in a gamma counter. The % lysis as represented by % $^{51}$Cr release was calculated as follows:

$$\frac{cpm\ experimental\ release\ -\ cpm\ spontaneous\ release}{cpm\ maximal\ release\ -\ cpm\ spontaneous\ release} \times 100$$

where spontaneous release = cpm released from target cells in medium alone and maximal release = cpm released from target cells in detergent. Spontaneous $^{51}$Cr release was usually less than 15% of maximal release. Results shown are the mean values of % $^{51}$Cr released from cells in 4 replicate wells.

Figure 1 depicts the % lysis of HIV-infected target cells by the treated and untreated PBL from seronegative individuals in the presence of the 110.4 x G19-4 heteroconjugate. As the figure indicates, the untreated PBL lysed the HIV-infected target cells in the presence of 20 to 200 ng/ml of the heteroconjugate. PBL pretreated with the anti-CD3 were even more cytotoxic than the untreated PBL.

Figure 2A depicts in table form the % lysis of HIV-infected cells vs. uninfected cells by anti-CD3-pretreated PBL from HIV seropositive and seronegative individuals in the presence of the 110.4 x G19-4 heteroconjugate or in the presence of mixtures of the individual 110.4 and G19-4 antibodies. PBL from HIV seropositive and seronegative donors were cultured with monomeric anti-CD3 (G19-4) on solid phase for 3 days and then cultured in anti-CD3-free medium for 24 hours. The PBL were then tested for cytotoxicity against HIV-infected and uninfected CEM cells at an E:T ratio of 50:1 in the presence of 200 ng/ml of the 110.4 x G19-4 heteroconjugate or a mixture of the 110.4 and G19-4 antibodies.

The data shows that a greater degree of lysis was mediated by the PBL in the presence of the heteroconjugate than in the presence of the mixture whereas there were negligible differences in the level of lysis of uninfected cells in the presence of the heteroconjugate vs. the antibody mixture. Thus, the PBL had been targeted by the heteroconjugate to kill the HIV-infected cells. The % lysis of HIV-infected or uninfected cells was not higher in the presence of the mixture of antibodies then in the absence of antibodies. Furthermore, Figure 2A indicates that PBL from asymptomatic HIV seropositive individuals are capable of lysing HIV-infected cells in the presence of the 110.4 x G19-4 heteroconjugate.

Figure 2B depicts the % lysis of HIV-infected cells by $CD8^+$ enriched PBL vs. unseparated seronegative PBL in the presence of the 110.4 x G19-4 heteroconjugate. Enrichment of the PBL was performed as follows: seronegative PBL were enriched for $CD8^+$ cells by negative selection as described by T. Lea et al., Scand. J. Immunol., 22, pp. 207-216 (1985). Briefly, PBL, activated for 3 days with anti-CD3 on solid phase, were treated with monoclonal antibodies to DR, CD20, CD16, CD11, CD4 and CDw14 to coat B cells, monocytes, LGLs (e.g., NK or K cells) and CD4 cells. The antibody-coated cells were then incubated with magnetic particles coated with sheep anti-mouse Ig (Dynal Inc., Fort Lee, N.J.) and were removed by a Dynal M-450 magnet. All the monoclonal antibodies used -- DR (HB10a), CD20 (IF5), CD16 (Fc2.2), CD11 (60.1), CD4 (G19-2) and CDw14 (f13) have been described [see, e.g., Leukocyte Typing, A. Bernard et al. (ed.s), Springer-Verlag, New York (1984); Leukocyte Typing II, E. Reinherz et al. (ed.s), Springer-Verlag, New York (1986); and Leukocyte Typing III, A.J. McMichael (ed.), Oxford University Press, Oxford (in press). The cell separation method resulted in an approximately three-fold enrichment for $CD8^+$ cells -- from 23% $CD8^+$ cells in the unseparated PBL population to 62% $CD8^+$ cells in the enriched population. The anti-CD3-treated unseparated and $CD8^+$-enriched cells were then incubated overnight in the absence of anti-CD3 before testing for cytotoxicity with the 110.4 x CD3 heteroconjugate.

Figure 2B indicates that CD3-activated $CD8^+$-enriched cells are more cytotoxic to the target cells then the unseparated cells, suggesting that the $CD8^+$ cells (i.e., cytotoxic T cells) within the PBL population are largely responsible for lysing the HIV-infected cells.

This example demonstrates, therefore, the ability of the 110.4 x G19-4 heteroconjugate of this invention to target both untreated and anti-CD3-treated PBL from either HIV seropositive or seronegative individuals to lyse HIV-infected cells. The data provided clearly indicates the utility of this approach for the treatment of HIV-infected individuals.

EXAMPLE 2

This example demonstrates the ability of a heteroconjugate of the invention, comprised of monoclonal antibody 110.4 cross-linked to a monoclonal antibody specific for the Fc receptor of certain effector cells (e.g., LGLs) to target PBL to lyse HIV-infected cells.

In this example, antibody 110.4, described above, is cross-linked by the methods described above to antibody Fc2, which is specific for the CD16 antigen identified as the Fc receptor expressed on LGLs and granulocytes. Fc2 has been prepared as described by J.A. Ledbetter et al., in Perspectives In Immunogenetics And Histocompatibility, supra. Furthermore, the hybridoma that produces the Fc2 antibody was deposited with the American Type Culture Collection prior to the filing of this application. The resulting heteroconjugate was designated 110.4 x Fc2.

Using the same $^{51}Cr$- release cytotoxicity assay described in Example 1, we tested the ability of this heteroconjugate to target seronegative PBL that were a) cultured for 2 days with or without human IL-2 (100 U/ml, Biotest Diagnostics, Fairfield, N.J.) or b) cultured overnight with or without $\beta$-IFN (300 U/ml, HEM, Maryland) to kill HIV-infected cells. The untreated and treated PBL were incubated for 4 hours at 37°C with $3 \times 10^3$ $^{51}Cr$-labeled HIV-infected CEM cells at an E:T ratio of 50:1 with varying concentrations of the heteroconjugate or an antibody mixture and % lysis determined as described in Example 1.

Figure 3 depicts the results of the assay. The PBL, both treated and untreated, were able to lyse the target cells in the presence of the heteroconjugate at heteroconjugate concentrations as low as 15 ng/ml. As shown in Figure 3A, the IL-2 activated cells were somewhat more cytotoxic toward the HIV-infected cells than the untreated PBL in the presence of the heteroconjugate. No appreciable lysis occurred in the presence of only the antibody mixture. Similarly, Figure 3B demonstrates that pretreatment of the PBL with $\beta$-IFN results in cells that are somewhat more lytic than untreated PBL in the presence of the heteroconjugate.

Figure 4 further demonstrates the ability of pretreatment with $\beta$-IFN to enhance the cytotoxicity of PBL in the presence of the 110.4 x Fc2 heteroconjugate. PBL from seronegative donors were isolated by ficoll-hypaque centrifugation, suspended at $1\times10^6$ cells/ml in RPMI-1640 medium supplemented with 10% heat-inactivated human serum and $\beta$-IFN at 0, 300 or 1000 U/ml and incubated at 37°C for 3 hours. The PBL were then washed, resuspended in RPMI-1640 medium supplemented with 15% heat-inactivated fetal calf serum prior to testing for cytotoxicity against $^{51}$Cr-labeled HIV-infected CEM cells. The assay was carried out as described above at an E:T cell ratio of 50:1 in a 4 hour assay in the presence of 200 ng/ml of the 110.4 x Fc2 heteroconjugate, a mixture of the individual antibodies of the heteroconjugate, the single antibodies or no antibodies. As the table in Figure 4 indicates, short term treatment of the PBL with $\beta$-IFN augments the cytotoxicity of the PBL in the presence of the heteroconjugate and thus, overnight treatment with $\beta$-IFN is not necessary.

Figure 5 depicts in table form the % lysis of HIV-infected cells by IL-2 pretreated PBL from HIV seropositive and seronegative individuals in the presence of the 110.4 x Fc2 heteroconjugate. PBL from HIV seropositive and seronegative donors were cultured for 2 days at 37°C with IL-2 (100 U/ml) and tested for cytotoxicity against HIV-infected and uninfected CEM cells at an E:T ratio of 50:1 in the presence of 200 ng/ml of the 110.4 x Fc2 heteroconjugate or a mixture of the two antibodies. This figure demonstrates the ability of PBL from seropositive (as well as seronegative) individuals to be targeted to lyse HIV-infected cells by the heteroconjugate of the invention. Augmented lysis of the uninfected cells in the presence of the heteroconjugate was not observed.

In order to determine which cells within the PBL population were responsible for the lysis seen with 110.4 x Fc2, we enriched PBL for CD16$^+$ cells by the method described in Example 1, except that the monoclonal antibodies used for coating the PBL were CD28 (9.3), CD5 (10.2), CD4 (G19-2), DR (HB10a), CD20 (IF5), and CDw14 (f13), all of which have been described in the Leukocyte Typing publications cited earlier. The PBL were enriched by this method from 17% CD16$^+$ cells in the unseparated PBL population to 66% CD16$^+$ cells in the enriched population. The unseparated and CD16$^+$-enriched cells were cultured for 2 days with IL-2 prior to testing for cytotoxicity at various E:T ratios in the presence of 200 ng/ml of 110.4 x Fc2. As Figure 6 indicates, the IL-2-treated PBL enriched for CD16$^+$ cells were more cytotoxic for HIV-infected cells than the unseparated cells, suggesting that CD16$^+$ LGL cells within the PBL population are targeted by this heteroconjugate for the lysis of the HIV-infected cells.

This example therefore demonstrates the ability of the heteroconjugates of this invention to target a second effector cell of the peripheral blood, i.e., LGL cells, for the killing of HIV-infected cells. In addition, we have demonstrated that pretreatment of the effector cells with either IL-2 or $\beta$-IFN enhances the ability of the heteroconjugates to target the effector cell to lyse the HIV-infected cell.

EXAMPLE 3

This example demonstrates the ability of another heteroconjugate of the invention, comprising a monoclonal antibody to a second HIV glycoprotein, gp41, cross-linked to monoclonal antibody, G19-4, to target PBL to lyse HIV-infected cells.

In this example, we utilized monoclonal antibody 41.1 as the HIV-specific antibody of the heteroconjugate. 41.1 is an antibody of subclass IgG$_1$ and reacts with an epitope on a highly conserved region of gp41 encoded by nucleotides 7178-7698 of LAV-1. The production of this monoclonal antibody is described in detail in L.H. Gosting et al., supra, and the hybridoma that produces the antibody has been deposited with the American Type Culture Collection prior to the filing of this application.

41.1 and G19-4 were cross-linked as described in Example 1 and the resulting heteroconjugate was designated 41.1 x G19-4. As in Example 1, the heteroconjugate was separated from free antibody by size exclusion chromatography using Sephacryl® S300 and the fraction with the highest binding activity to both gp41 on HIV-infected cells and to CD3 antigen on human PBL was then tested in the cytotoxicity assay described in Example 1. Briefly, the effector cells were PBL from seronegative donors that had been cultured for three days with anti-CD3 on solid phase followed by overnight incubation in anti-CD3-free medium. The effector cells were then incubated for 4 hours at 37°C with $3\times10^3$ $^{51}$Cr-labeled target CEM cells as described in Example 1 at a E:T ratio of 50:1 in the presence of either a) the 41.1 x G19-4 heteroconjugate; b) a 41.1 plus G19-4 antibody mixture; c) 41.1 alone or d) G19-4 alone and % lysis of the CEM cells determined. The results of this assay are depicted in Figure 7. The figure indicates the ability of the 41.1 x G19-4 heteroconjugate to target PBL to kill HIV-infected cells. The PBL were stimulated to lyse the HIV-infected cells in the presence of approximately 50 to 200 ng/ml of the heteroconjugate. Lysis in the presence of the antibody mixture or the individual antibodies alone was negligible.

Thus, the heteroconjugate approach of this invention for killing HIV-infected cells can employ any of a number of HIV-specific antibodies cross-linked to effector cell-specific antibodies. In fact, the 41.1 monoclonal antibody is particularly useful in this approach because it was reactive with a majority of the isolates of HIV tested (10 out of 13), the isolates having been derived from various geographical areas of the world.

Hybridomas prepared by the processes described herein are exemplified by cultures deposited in the American Type Culture Collection , Rockville, Maryland. The cultures were deposited on September 15, 1987, and are there identified as follows:

Hybridoma G19-4:     ATCC No. HB9536
Hybridoma Fc2:       ATCC No. HB9535
Hybridoma 41.1:      ATCC No. HB9534

While we have hereinbefore presented a number of embodiments of this invention, it should be understood that the scope of this invention is to be defined by the claims appended hereto rather than by the specific embodiments which have been presented hereinbefore by way of example.

**Claims**
**Claims for the following Contracting States : AT, BE, CH, DE, FR, GB, IT, LI, LU, NL, SE**

1. An antibody heteroconjugate comprising at least one antibody reactive with an HIV antigen expressed on the surface of an HIV-infected cell cross-linked to at least one antibody reactive with an effector cell of the peripheral blood capable of killing an HIV-infected cell.

2. The antibody heteroconjugate of claim 1, wherein the HIV antigen is one found on an HIV envelope glycoprotein.

3. The antibody heteroconjugate of claim 1, wherein the effector cell is selected from T lymphocytes, large granular lymphocytes, granulocytes, monocytes and macrophages.

4. The antibody heteroconjugate of claim 1, wherein the effector cell-reactive antibody is selected from antibodies reactive with the T cell receptor on T lymphocytes and an Fc receptor on leukocytes.

5. The antibody heteroconjugate of claim 1, wherein the effector cell-reactive antibody is an antibody to the CD3 antigen on the T cell receptor of T lymphocytes.

6. The antibody heteroconjugate of claim 1, wherein the effector cell-reactive antibody is an antibody to the CD16 Fc receptor of large granular lymphocytes and granulocytes.

7. The antibody heteroconjugate of claim 1, wherein the antibodies are antibody fragments selected from Fab and F(ab')$_2$ fragments.

8. The antibody heteroconjugate of claim 1, wherein the antibodies are chimeric antibodies.

9. An antibody heteroconjugate comprising a first antibody reactive with an HIV antigen expressed on the surface of an HIV-infected cell cross-linked to a second antibody reactive with an effector cell of the peripheral blood capable of killing an HIV-infected cell.

10. The antibody heteroconjugate of claim 9 selected from 110.4 x G19-4, 110.4 x Fc2 and 41.1 x G19-4.

11. The use of effector cells of the peripheral blood in the presence of at least one antibody-heteroconjugate according to one of claims 1 to 10 for preparing a pharmaceutical composition for killing HIV infected cells.

12. The use of claim 11, wherein the effector cells are selected from peripheral blood lymphocytes, granulocytes, monocytes and macrophages.

13. The use of claim 11, wherein the effector cells are obtained from HIV seropositive or seronegative individuals.

14. The use of claim 11, wherein the effector cells are pretreated with a compound selected from interleukin-2, $\beta$-interferon, $\alpha$-interferon and $\gamma$-interferon.

**15.** The use of claim 11, wherein the effector cells are pretreated with an antibody specific for the effector cells.

**16.** The use of claim 15, wherein the antibody is one that stimulates the lytic mechanism of the effector cells.

**17.** The use of claim 16, wherein the antibody is an anti-CD3 antibody.

**18.** The use of claim 11 or 14, wherein the effector cells are cytotoxic T lymphocytes and the antibody heteroconjugate is selected from 110.4 x G19-4 and 41.1 x G19-4.

**19.** The use of claim 11 or 14, wherein the effector cells are large granular lymphocytes and the antibody heteroconjugate is 110.4 x Fc2.

**20.** A pharmaceutically acceptable composition useful in the treatment of HIV infections which comprises a pharmaceutically effective amount of at least one antibody heteroconjugate according to one of claims 1 to 10.

**21.** The use of a pharmaceutically effective amount of at least one antibody heteroconjugate according to anyone of claims 1 to 10 for preparing a pharmaceutical composition for treating HIV infections.

**22.** The use of claim 21, wherein additionally a pharmaceutically effective amount of a compound selected from interleukin-2, $\beta$-interferon, $\alpha$-interferon and $\gamma$-interferon is employed.

**23.** The use of effector cells of the peripheral blood capable of killing HIV-infected cells which effector cells were treated with at least one antibody heteroconjugate according to anyone of claims 1 to 10 in vitro and of said heteroconjugate for preparing a pharmaceutical composition for treating HIV infections.

**24.** The use of claim 23, wherein the effector cell is selected from T lymphocytes, large granular lymphocytes, granulocytes, monocytes and macrophages.

**25.** The use of claim 23, wherein the effector cells are pretreated with a compound selected from interleukin-2, $\beta$-interferon, $\alpha$-interferon and $\gamma$-interferon.

**26.** The use of claim 23, wherein the effector cells are pretreated with an antibody specific for the effector cells.

**27.** The use of claim 26, wherein the antibody is one that stimulates the lytic mechanism of the effector cell.

**28.** The use of claim 27, wherein the antibody is an anti-CD3 antibody.

**29.** A process for preparing an antibody heteroconjugate having at least one first antibody reactive with an HIV antigen expressed on the surface of an HIV-infected cell cross-linked to at least one second antibody reactive with an effector cell of the peripheral blood capable of killing an HIV-infected cell, comprising the step of reacting said first and second antibody with a heterobifunctional cross-linking agent.

**30.** The process of claim 29, wherein the cross-linking agent is N-succinimidyl-3-(2-pyridylthio)-propionate (SPDP) or maleimidobutryloxysuccinimide (GMBS).

**31.** The process of claim 29, wherein said first antibody is 110.4 and said second antibody is G19-4; said first antibody is 110.4 and said second antibody is Fc2; or said first antibody is 41.1 and said second antibody is G19-4.

13

**Claims for the following Contracting State : ES**

1. A process for preparing an antibody heteroconjugate having at least one first antibody reactive with an HIV antigen expressed on the surface of an HIV-infected cell cross-linked to at least one second antibody reactive with an effector cell of the peripheral blood capable of killing an HIV-infected cell, comprising the step of reacting said first and second antibody with a heterobifunctional cross-linking agent.

2. The process of claim 1, wherein the HIV antigen is one found on an HIV envelope glycoprotein.

3. The process of claim 1, wherein the effector cell is selected from T lymphocytes, large granular lymphocytes, granulocytes, monocytes and macrophages.

4. The process of claim 1, wherein the effector cell-reactive antibody is selected from antibodies reactive with the T cell receptor on T lymphocytes and an Fc receptor on leukocytes.

5. The process of claim 1, wherein the effector cell-reactive antibody is an antibody to the CD3 antigen on the T cell receptor of T lymphocytes.

6. The process of claim 1, wherein the effector cell-reactive antibody is an antibody to the CD16 Fc receptor of large granular lymphocytes and granulocytes.

7. The process of claim 1, wherein the antibodies are antibody fragments selected from Fab and F(ab')$_2$ fragments.

8. The process of claim 1, wherein the antibodies are chimeric antibodies.

9. The process of one of claims 1 to 8, wherein the antibody heteroconjugate comprises a first antibody reactive with an HIV antigen expressed on the surface of an HIV-infected cell cross-linked to a second antibody reactive with an effector cell of the peripheral blood capable of killing an HIV-infected cell.

10. The process of one of the claims 1 to 9, wherein said first antibody is 110.4 and said second antibody is G19-4;
    said first antibody is 110.4 and said second antibody is Fc2; or
    said first antibody is 41.1 and said second antibody is G19-4.

11. The process of one of the claims 1 to 10, wherein the cross-linking agent is N-succinimidyl-3(2-pyrridylthio)-propionate (SPDP) or maleimidobutryloxysuccinimide (GMBS).

12. The use of effector cells of the peripheral blood in the presence of at least one antibody heteroconjugate prepared according to one of claim 1 to 11 for preparing a pharmaceutical composition for killing HIV-infected cells.

13. The use of claim 12, wherein the effector cells are selected from blood lymphocytes, granulocytes, monocytes and macrophages.

14. The use of claim 12, wherein the effector cells are obtained from HIV seropositive or seronegative individuals.

15. The use of claim 12, wherein the effector cells are pretreated with a compound selected from interleukin-2, $\beta$-interferon, $\alpha$-interferon and $\gamma$-interferon.

16. The use of claim 12, wherein the effector cells are pretreated with an antibody specific for the effector cells.

17. The use of claim 16, wherein the antibody is one that stimulates the lytic mechanism of the effector cells.

EP 0 308 936 B1

**18.** The use of claim 17, wherein the antibody is an anti-CD3 antibody.

**19.** The use of claim 12 or 15, wherein the effector cells are cytotoxic T lymphocytes and the antibody heteroconjugate is selected from 110.4 x G19-4 and 41.1 x G19-4.

**20.** The use of claim 12 or 15, wherein the effector cells are large granular lymphocytes and the antibody heteroconjugate is 110.4 x Fc2.

**21.** The use of a pharmaceutically effective amount of at least one antibody heteroconjugate prepared according to anyone of claims 1 to 11 for preparing a pharmaceutical composition for treating HIV infections.

**22.** The use of claim 21, wherein additionally a pharmaceutically effective amount of a compound selected from interleukin-2, $\beta$-interferon, $\alpha$-interferon and $\gamma$-interferon is employed.

**23.** The use of effector cells of the peripheral blood capable of killing HIV-infected cells which effector cells were treated with at least one antibody heteroconjugate prepared according to anyone of claims 1 to 11 in vitro and of said heteroconjugate for preparing a pharmaceutical composition for treating HIV infections.

**24.** The use of claim 23, wherein the effector cell is selected from T lymphocytes, large granular lymphocytes, granulocytes, monocytes and macrophages.

**25.** The use of claim 23, wherein the effector cells are pretreated with a compound selected from interleukin-2, $\beta$-interferon, $\alpha$-interferon and $\gamma$-interferon.

**26.** The use of claim 23, wherein the effector cells are pretreated with in antibody specific for the effector cells.

**27.** The use of claim 26, wherein the antibody is one that stimulates the lytic mechanism of the effector cell.

**28.** The use of claim 27, wherein the antibody is an anti-CD3 antibody.

**29.** A process for preparing a pharmaceutically accceptable composition useful in the treatment of HIV-infections which comprises providing a pharmaceutically effective amount of at least one antibody heteroconjugate prepared according to one of claims 1 to 11.

**30.** The process of claim 29 wherein additionally a pharmaceutically effective amount of a compound selected from interleukin-2, $\beta$-interferon, $\alpha$-interferon and $\gamma$-interferon is employed.

**31.** A process for preparing a pharmaceutical composition for killing HIV infected cells which comprises providing pharmaceutically effective amounts of effector cells and antibody heteroconjugates as defined in one of the claims 12 to 20.

**32.** A process for preparing a pharmaceutical composition for treating HIV-infections, which comprises providing pharmaceutically effective amounts of effector cells and antibody heteroconjugates as defined in one of claims 23 to 28.

**Claims for the following Contracting State : GR**

**1.** An antibody heteroconjugate comprising at least one antibody reactive with an HIV antigen expressed on the surface of an HIV-infected cell cross-linked to at least one antibody reactive with an effector cell of the peripheral blood capable of killing an HIV-infected cell.

**2.** The antibody heteroconjugate of claim 1, wherein the HIV antigen is one found on an HIV envelope glycoprotein.

15

**3.** The antibody heteroconjugate of claim 1, wherein the effector cell is selected from T lymphocytes, large granular lymphocytes, granulocytes, monocytes and macrophages.

**4.** The antibody heteroconjugate of claim 1, wherein the effector cell-reactive antibody is selected from antibodies reactive with the T cell receptor on T lymphocytes and an Fc receptor on leukocytes.

**5.** The antibody heteroconjugate of claim 1, wherein the effector cell-reactive antibody is an antibody to the CD3 antigen on the T cell receptor of T lymphocytes.

**6.** The antibody heteroconjugate of claim 1, wherein the effector cell-reactive antibody is an antibody to the CD16 Fc receptor of large granular lymphocytes and granulocytes.

**7.** The antibody heteroconjugate of claim 1, wherein the antibodies are antibody fragments selected from Fab and F(ab')$_2$ fragments.

**8.** The antibody heteroconjugate of claim 1, wherein the antibodies are chimeric antibodies.

**9.** An antibody heteroconjugate comprising a first antibody reactive with an HIV antigen expressed on the surface of an HIV-infected cell cross-linked to a second antibody reactive with an effector cell of the peripheral blood capable of killing an HIV-infected cell.

**10.** The antibody heteroconjugate of claim 9 selected from 110.4 x G19-4, 110.4 x Fc2 and 41.1 x G19-4.

**11.** The use of effector cells of the peripheral blood in the presence of at least one antibody-heteroconjugate according to one of claims 1 to 10 for preparing a pharmaceutical composition for killing HIV infected cells.

**12.** The use of claim 11, wherein the effector cells are selected from peripheral blood lymphocytes, granulocytes, monocytes and macrophages.

**13.** The use of claim 11, wherein the effector cells are obtained from HIV seropositive or seronegative individuals.

**14.** The use of claim 11, wherein the effector cells are pretreated with a compound selected from interleukin-2, $\beta$-interferon, $\alpha$-interferon and $\gamma$-interferon.

**15.** The use of claim 11, wherein the effector cells are pretreated with an antibody specific for the effector cells.

**16.** The use of claim 15, wherein the antibody is one that stimulates the lytic mechanism of the effector cells.

**17.** The use of claim 16, wherein the antibody is an anti-CD3 antibody.

**18.** The use of claim 11 or 14, wherein the effector cells are cytotoxic T lymphocytes and the antibody heteroconjugate is selected from 110.4 x G19-4 and 41.1 x G19-4.

**19.** The use of claim 11 or 14, wherein the effector cells are large granular lymphocytes and the antibody heteroconjugate in 110.4 x Fc2.

**20.** A process for preparing a pharmaceutically acceptable composition useful in the treatment of HIV infections which comprises providing a pharmaceutically effective amount of at least one antibody heteroconjugate according to one of claims 1 to 10.

**21.** The use of a pharmaceutically effective amount of at least one antibody heteroconjugate according to anyone of claims 1 to 10 for preparing a pharmaceutical composition for treating HIV infections.

**22.** The use of claim 21, wherein additionally a pharmaceutically effective amount of a compound selected from interleukin-2, $\beta$-interferon, $\alpha$-interferon and $\gamma$-interferon is employed.

**23.** The use of effector cells of the peripheral blood capable of killing HIV-infected cells which effector cells were treated with at least one antibody heteroconjugate according to anyone of claims 1 to 10 <u>in vitro</u> and of said heteroconjugate for preparing a pharmaceutical composition for treating HIV infections.

**24.** The use of claim 23, wherein the effector cell is selected from T lymphocytes, large granular lymphocytes, granulocytes, monocytes and macrophages.

**25.** The use of claim 23, wherein the effector cells are pretreated with a compound selected from interleukin-2, $\beta$-interferon, $\alpha$-interferon and $\gamma$-interferon.

**26.** The use of claim 23, wherein the effector cells are pretreated with an antibody specific for the effector cells.

**27.** The use of claim 26, wherein the antibody is one that stimulates the lytic mechanism of the effector cell.

**28.** The use of claim 27, wherein the antibody is an anti-CD3 antibody.

**29.** A process for preparing an antibody heteroconjugate having at least one first antibody reactive with an HIV antigen expressed on the surface of an HIV-infected cell cross-linked to at least one second antibody reactive with an effector cell of the peripheral blood capable of killing an HIV-infected cell, comprising the step of reacting said first and second antibody with a heterobifunctional cross-linking agent.

**30.** The process of claim 29, wherein the cross-linking agent is N-succinimidyl-3-(2-pyridylthio)-propionate (SPDP) or maleimidobutryloxysuccinimide (GMBS).

**31.** The process of claim 29, wherein said first antibody is 110.4 and said second antibody is G19-4;
said first antibody is 110.4 and said second antibody is Fc2; or
said first antibody is 41.1 and said second antibody is G19-4.

**Patentansprüche**
**Patentansprüche für folgende Vertragsstaaten : AT, BE, CH, DE, FR, GB, IT, LI, LU, NL, SE**

**1.** Antikörper-Heterokonjugat, umfassend wenigstens einen Antikörper, der mit einem HIV-Antigen reagiert, das auf der Oberfläche einer HIV-infizierten Zelle exprimiert wird, der mit wenigstens einem Antikörper verknüpft ist, der mit einer Effektorzelle des peripheren Blutsystems reagiert, die zur Abtötung einer HIV-infizierten Zelle befähigt ist.

**2.** Antikörper-Heterokonjugat nach Anspruch 1, wobei das HIV-Antigen auf einem HIV-Envelope-Glykoprotein zu finden ist.

**3.** Antikörper-Heterokonjugat nach Anspruch 1, worin die Effektorzelle ausgewählt ist unter T-Lymphocyten, großen granulären Lymphocyten, Granulocyten, Monocyten und Makrophagen.

**4.** Antikörper-Heterokonjugat nach Anspruch 1, worin der mit der Effektorzelle reagierende Antikörper ausgewählt ist unter Antikörpern, die mit dem T-Zellrezeptor auf T-Lymphocyten und einem Fc-Rezeptor auf Leukocyten reagieren.

**5.** Antikörper-Heterokonjugat nach Anspruch 1, worin der mit der Effektorzelle reagierende Antikörper ein Antikörper für das CD3 Antigen auf dem T-Zellrezeptor von T-Lymphocyten ist.

**6.** Antikörper-Heterokonjugat nach Anspruch 1, worin der mit der Effektorzelle reagierende Antikörper ein Antikörper für den CD16 Fc-Rezeptor von großen granulären Lymphocyten und Granulocyten ist.

17

**7.** Antikörper-Heterokonjugat nach Anspruch 1, worin die Antikörper und Antikörperfragmente ausgewählt sind unter Fab und F(ab')$_2$-Fragmenten.

**8.** Antikörper-Heterokonjugat nach Anspruch 1, worin die Antikörper chimäre Antikörper sind.

**9.** Antikörper-Heterokonjugat, umfassend einen ersten Antikörper, der mit einem HIV-Antigen reagiert, das auf der Oberfläche einer HIV-infizierten Zelle exprimiert wird, der mit einem zweiten Antikörper verbunden ist, welcher mit einer Effektorzelle des peripheren Blutsystems reagiert, die zur Abtötung einer HIV-infizierten Zelle befähigt ist.

**10.** Antikörper-Heterokonjugat nach Anspruch 9, ausgewählt unter 110.4 x G19-4, 110.4 x Fc2 und 41.1 x G19-4.

**11.** Verwendung von Effektorzellen des peripheren Blutsystems in Gegenwart von wenigstens einem Antikörper-Heterokonjugat gemäß einem der Ansprüche 1 bis 10 zur Herstellung eines pharmazeutischen Mittels zur Abtötung HIV-infizierter Zellen.

**12.** Verwendung nach Anspruch 11, worin die Effektorzellen ausgewählt sind unter Peripherblut-Lymphocyten, Granulocyten, Monocyten und Makrophagen.

**13.** Verwendung nach Anspruch 11, worin die Effektorzellen erhalten wurden aus HIV-seropositiven oder -seronegativen Individuen.

**14.** Verwendung nach Anspruch 11, worin die Effektorzellen mit einer Verbindung vorbehandelt wurden, die ausgewählt ist unter Interleukin-2, $\beta$-Interferon, $\alpha$-Interferon und $\gamma$-Interferon.

**15.** Verwendung nach Anspruch 11, worin die Effektorzellen mit einem Effektorzellen-spezifischen Antikörper vorbehandelt wurden.

**16.** Verwendung nach Anspruch 15, worin der Antikörper den lytischen Mechanismus der Effektorzellen stimuliert.

**17.** Verwendung nach Anspruch 16, worin der Antikörper ein Anti-CD3-Antikörper ist.

**18.** Verwendung nach Anspruch 11 oder 14, worin die Effektorzellen cytotoxische T-Lymphocyten sind und das Antikörper-Heterokonjugat ausgewählt ist unter 110.4 x G19-4 und 41.1 x G19-4.

**19.** Verwendung nach Anspruch 11 oder 14, worin die Effektorzellen große granuläre Lymphocyten sind und das Antikörper-Heterokonjugat 110.4 x Fc2 ist.

**20.** Pharmazeutisch verträgliche Zusammensetzung zur Behandlung von HIV-Infektionen, umfassend eine pharmazeutisch wirksame Menge wenigstens eines Antikörper-Heterokonjugats gemäß einem der Ansprüche 1 bis 10.

**21.** Verwendung einer pharmazeutisch wirksamen Menge wenigstens eines Antikörper-Heterokonjugats gemäß einem der Ansprüche 1 bis 10 zur Herstellung eines pharmazeutischen Mittels zur Behandlung von HIV-Infektionen.

**22.** Verwendung nach Anspruch 21, worin zusätzlich eine pharmazeutisch wirksame Menge wenigstens einer Verbindung verwendet wird, die ausgewählt ist unter Interleukin-2, $\beta$-Interferon, $\alpha$-Interferon und $\gamma$-Interferon.

**23.** Verwendung von Effektorzellen des peripheren Blutsystems, die zur Abtötung von HIV-infizierten Zellen befähigt sind, wobei die Effektorzellen mit wenigstens einem Antikörper-Heterokonjugat gemäß einem der Ansprüche 1 bis 10 in vitro behandelt wurden, und des Heterokonjugats zur Herstellung eines pharmazeutischen Mittels zur Behandlung von HIV-Infektionen.

**24.** Verwendung nach Anspruch 23, worin die Effektorzelle ausgewählt ist unter T-Lymphocyten, großen granulären Lymphocyten, Granulocyten, Monocyten und Makrophagen.

**25.** Verwendung nach Anspruch 23, worin die Effektorzellen vorbehandelt wurden mit einer Verbindung, die ausgewählt ist unter Interleukin-2, $\beta$-Interferon, $\alpha$-Interferon und $\gamma$-Interferon.

**26.** Verwendung nach Anspruch 23, worin die Effektorzellen mit einem Effektorzellen-spezifischen Antikörper vorbehandelt wurden.

**27.** Verwendung nach Anspruch 26, worin der Antikörper den lytischen Mechanismus der Effektorzelle stimuliert.

**28.** Verwendung nach Anspruch 27, worin der Antikörper ein Anti-CD3-Antikörper ist.

**29.** Verfahren zur Herstellung eines Antikörper-Heterokonjugats, umfassend wenigstens einen ersten Antikörper, der mit einem HIV-Antigen reagiert, das auf der Oberfläche einer HIV-infizierten Zelle exprimiert wird, der mit wenigstens einem zweiten Antikörper vernetzt ist, welcher mit einer Effektorzelle des peripheren Blutsystems reagiert, die zur Abtötung einer HIV-infizierten Zelle befähigt ist, wobei man den ersten und den zweiten Antikörper mit einem heterobifunktionellen Vernetzungsmittel umsetzt.

**30.** Verfahren nach Anspruch 29, wobei das Vernetzungsmittel N-Succinimidyl-3-(2-pyridylthio)-propionat (SPDP) oder Maleimidobutryloxysuccinimid (GMBS) ist.

**31.** Verfahren nach Anspruch 29, wobei der erste Antikörper 110.4 und der zweite Antikörper G19-4 ist; oder
der erste Antikörper 110.4 und der zweite Antikörper Fc2 ist; oder
der erste Antikörper 41.4 und der zweite Antikörper G19-4 ist.

**Patentansprüche für folgenden Vertragsstaat : ES**

**1.** Verfahren zur Herstellung eines Antikörper-Heterokonjugats, umfassend wenigstens einen ersten Antikörper, der mit einem HIV-Antigen reagiert, das auf der Oberfläche einer HIV-infizierten Zelle exprimiert wird, der mit wenigstens einem zweiten Antikörper vernetzt ist, welcher mit einer Effektorzelle des peripheren Blutsystems reagiert, die zur Abtötung einer HIV-infizierten Zelle befähigt ist, wobei man den ersten und den zweiten Antikörper mit einem heterobifunktionellen Vernetzungsmittel umsetzt.

**2.** Verfahren nach Anspruch 1, wobei das HIV-Antigen auf einem HIV-Envelope-Glykoprotein zu finden ist.

**3.** Verfahren nach Anspruch 1, worin die Effektorzelle ausgewählt ist unter T-Lymphocyten, großen granulären Lymphocyten, Granulocyten, Monocyten und Makrophagen.

**4.** Verfahren nach Anspruch 1, worin der mit der Effektorzelle reagierende Antikörper ausgewählt ist unter Antikörpern, die mit dem T-Zellrezeptor auf T-Lymphocyten und einem Fc-Rezeptor auf Leukocyten reagieren.

**5.** Verfahren nach Anspruch 1, worin der mit der Effektorzelle reagierende Antikörper ein Antikörper für das CD3 Antigen auf dem T-Zellrezeptor von T-Lymphocyten ist.

**6.** Verfahren nach Anspruch 1, worin der mit der Effektorzelle reagierende Antikörper ein Antikörper für den CD16 Fc-Rezeptor von großen granulären Lymphocyten und Granulocyten ist.

**7.** Verfahren nach Anspruch 1, worin die Antikörper und Antikörperfragmente ausgewählt sind unter Fab und F(ab')$_2$-Fragmenten.

**8.** Verfahren nach Anspruch 1, worin die Antikörper chimäre Antikörper sind.

**9.** Verfahren nach einem der Ansprüche 1 bis 8, worin das Antikörper-Heterokonjugat einen ersten Antikörper umfaßt, der mit einem HIV-Antigen reagiert, das auf der Oberfläche einer HIV-infizierten Zelle exprimiert wird, der mit einem zweiten Antikörper vernetzt ist, welcher mit einer Effektorzelle des peripheren Blutsystems reagiert, die zur Abtötung einer HIV-infizierten Zelle befähigt ist.

10. Verfahren nach einem der Ansprüche 1 bis 9, wobei der erste Antikörper 110.4 und der zweite Antikörper G19-4 ist; oder
der erste Antikörper 110.4 und der zweite Antikörper Fc2 ist; oder
der erste Antikörper 41.4 und der zweite Antikörper G19-4 ist.

11. Verfahren nach einem der Ansprüche 1 bis 10, worin das Vernetzungsmittel ausgewählt ist unter N-Succinimidyl-3-(2-pyridylthio)-propionat (SPDP) oder Maleimidobutryloxysuccinimid (GMBS).

12. Verwendung von Effektorzellen des peripheren Blutsystems in Gegenwart eines Antikörper-Heterokonjugats, hergestellt gemäß einem der Ansprüche 1 bis 11, zur Herstellung eines pharmazeutischen Mittels zur Abtötung von HIV-infizierten Zellen.

13. Verwendung nach Anspruch 12, worin die Effektorzellen ausgewählt sind unter Blutlymphocyten, Granulocyten, Monocyten und Makrophagen.

14. Verwendung nach Anspruch 12, worin die Effektorzellen von HIV-seropositiven oder -seronegativen Individuen erhalten wurden.

15. Verwendung nach Anspruch 12, worin die Effektorzellen mit einer Verbindung vorbehandelt wurden, die ausgewählt ist unter Interleukin-2, $\beta$-Interferon, $\alpha$-Interferon und $\gamma$-Interferon.

16. Verwendung nach Anspruch 12, worin die Effektorzellen mit einem Effektorzellen-spezifischen Antikörper vorbehandelt wurden.

17. Verwendung nach Anspruch 16, worin der Antikörper den lytischen Mechanismus der Effektorzellen stimuliert.

18. Verwendung nach Anspruch 17, worin der Antikörper ein Anti-CD3-Antikörper ist.

19. Verwendung nach Anspruch 12 oder 15, worin die Effektorzellen cytotoxische T-Lymphocyten sind und das Antikörper-Heterokonjugat ausgewählt ist unter 110.4 x G19-4 und 41.1 x G19-4.

20. Verwendung nach Anspruch 12 oder 15, worin die Effektorzellen große granuläre Lymphocyten sind und das Antikörper-Heterokonjugat 110.4 x Fc2 ist.

21. Verwendung einer pharmazeutisch wirksamen Menge wenigstens eines Antikörper-Heterokonjugats, hergestellt gemäß einem der Ansprüche 1 bis 11, zur Herstellung eines pharmazeutischen Mittels zur Behandlung von HIV-Infektionen.

22. Verwendung nach Anspruch 21, worin zusätzlich eine pharmazeutisch wirksame Menge einer Verbindung verwendet wird, die ausgewählt ist unter Interleukin-2, $\beta$-Interferon, $\alpha$-Interferon und $\gamma$-Interferon.

23. Verwendung von Effektorzellen des peripheren Blutsystems, die zur Abtötung von HIV-infizierten Zellen befähigt sind, wobei die Effektorzellen mit wenigstens einem Antikörper-Heterokonjugat, hergestellt gemäß einem der Ansprüche 1 bis 11, in vitro vorbehandelt wurden, und des Heterokonjugats zur Herstellung eines pharmazeutischen Mittels zur Behandlung von HIV-Infektionen.

24. Verwendung nach Anspruch 23, worin die Effektorzelle ausgewählt ist unter T-Lymphocyten, großen granulären Lymphocyten, Granulocyten, Monocyten und Makrophagen.

25. Verwendung nach Anspruch 23, worin die Effektorzellen vorbehandelt wurden mit einer Verbindung, die ausgewählt ist unter Interleukin-2, $\beta$-Interferon, $\alpha$-Interferon und $\gamma$-Interferon.

26. Verwendung nach Anspruch 23, worin die Effektorzellen mit einem Effektorzellen-spezifischen Antikörper vorbehandelt wurden.

**27.** Verwendung nach Anspruch 26, worin der Antikörper den lytischen Mechanismus der Effektorzelle stimuliert.

**28.** Verwendung nach Anspruch 27, worin der Antikörper ein Anti-CD3-Antikörper ist.

**29.** Verfahren zur Herstellung eines pharmazeutisch verträglichen Mittels zur Behandlung von HIV-Infektionen, wobei man eine pharmazeutisch wirksame Menge wenigstens eines Antikörper-Heterokonjugats bereitstellt, das gemäß einem der Ansprüche 1 bis 11 hergestellt wurde.

**30.** Verfahren nach Anspruch 29, wobei zusätzlich eine pharmazeutisch wirksame Menge einer Verbindung verwendet wird, die ausgewählt ist unter Interleukin-2, $\beta$-Interferon, $\alpha$-Interferon und $\gamma$-Interferon.

**31.** Verfahren zur Herstellung eines pharmazeutischen Mittels zur Abtötung von HIV-infizierten Zellen, wobei man pharmazeutisch wirksame Mengen von Effektorzellen und Antikörper-Heterokonjugaten gemäß einem der Ansprüche 12 bis 20 bereitstellt.

**32.** Verfahren zur Herstellung eines pharmazeutischen Mittels zur Behandlung von HIV-Infektionen, wobei man pharmazeutisch wirksame Mengen von Effektorzellen und Antikörper-Heterokonjugaten gemäß einem der Ansprüche 23 bis 28 bereitstellt.

**Patentansprüche für folgenden Vertragsstaat : GR**

**1.** Antikörper-Heterokonjugat, umfassend wenigstens einen Antikörper, der mit einem HIV-Antigen reagiert, das auf der Oberfläche einer HIV-infizierten Zelle exprimiert wird, der mit wenigstens einem Antikörper verknüpft ist, der mit einer Effektorzelle des peripheren Blutsystems reagiert, die zur Abtötung einer HIV-infizierten Zelle befähigt ist.

**2.** Antikörper-Heterokonjugat nach Anspruch 1, wobei das HIV-Antigen auf einem HIV-Envelope-Glykoprotein zu finden ist.

**3.** Antikörper-Heterokonjugat nach Anspruch 1, worin die Effektorzelle ausgewählt ist unter T-Lymphocyten, großen granulären Lymphocyten, Granulocyten, Monocyten und Makrophagen.

**4.** Antikörper-Heterokonjugat nach Anspruch 1, worin der mit der Effektorzelle reagierende Antikörper ausgewählt ist unter Antikörpern, die mit dem T-Zellrezeptor auf T-Lymphocyten und einem Fc-Rezeptor auf Leukocyten reagieren.

**5.** Antikörper-Heterokonjugat nach Anspruch 1, worin der mit der Effektorzelle reagierende Antikörper ein Antikörper für das CD3 Antigen auf dem T-Zellrezeptor von T-Lymphocyten ist.

**6.** Antikörper-Heterokonjugat nach Anspruch 1, worin der mit der Effektorzelle reagierende Antikörper ein Antikörper für den CD16 Fc-Rezeptor von großen granulären Lymphocyten und Granulocyten ist.

**7.** Antikörper-Heterokonjugat nach Anspruch 1, worin die Antikörper und Antikörperfragmente ausgewählt sind unter Fab und F(ab')$_2$-Fragmenten.

**8.** Antikörper-Heterokonjugat nach Anspruch 1, worin die Antikörper chimäre Antikörper sind.

**9.** Antikörper-Heterokonjugat, umfassend einen ersten Antikörper, der mit einem HIV-Antigen reagiert, das auf der Oberfläche einer HIV-infizierten Zelle exprimiert wird, der mit einem zweiten Antikörper verbunden ist, welcher mit einer Effektorzelle des peripheren Blutsystems reagiert, die zur Abtötung einer HIV-infizierten Zelle befähigt ist.

**10.** Antikörper-Heterokonjugat nach Anspruch 9, ausgewählt unter 110.4 x G19-4, 110.4 x Fc2 und 41.1 x G19-4.

**11.** Verwendung von Effektorzellen des peripheren Blutsystems in Gegenwart von wenigstens einem Antikörper-Heterokonjugat gemäß einem der Ansprüche 1 bis 10 zur Herstellung eines pharmazeutischen Mittels zur Abtötung HIV-infizierter Zellen.

12. Verwendung nach Anspruch 11, worin die Effektorzellen ausgewählt sind unter Peripherblut-Lymphocyten, Granulocyten, Monocyten und Makrophagen.

13. Verwendung nach Anspruch 11, worin die Effektorzellen erhalten wurden aus HIV-seropositiven oder -seronegativen Individuen.

14. Verwendung nach Anspruch 11, worin die Effektorzellen mit einer Verbindung vorbehandelt wurden, die ausgewählt ist unter Interleukin-2, $\beta$-Interferon, $\alpha$-Interferon und $\gamma$-Interferon.

15. Verwendung nach Anspruch 11, worin die Effektorzellen mit einem Effektorzellen-spezifischen Antikörper vorbehandelt wurden.

16. Verwendung nach Anspruch 15, worin der Antikörper den lytischen Mechanismus der Effektorzellen stimuliert.

17. Verwendung nach Anspruch 16, worin der Antikörper ein Anti-CD3-Antikörper ist.

18. Verwendung nach Anspruch 11 oder 14, worin die Effektorzellen cytotoxische T-Lymphocyten sind und das Antikörper-Heterokonjugat ausgewählt ist unter 110.4 x G19-4 und 41.1 x G19-4.

19. Verwendung nach Anspruch 11 oder 14, worin die Effektorzellen große granuläre Lymphocyten sind und das Antikörper-Heterokonjugat 110.4 x Fc2 ist.

20. Verfahren zur Herstellung einer pharmazeutisch verträglichen Zusammensetzung, für die Behandlung von HIV-Infektionen, wobei man eine pharmazeutisch wirksame Menge wenigstens eines Antikörper-Heterokonjugats gemäß einem der Ansprüche 1 bis 10 bereitstellt.

21. Verwendung einer pharmazeutisch wirksamen Menge wenigstens eines Antikörper-Heterokonjugats gemäß einem der Ansprüche 1 bis 10 zur Herstellung eines pharmazeutischen Mittels zur Behandlung von HIV-Infektionen.

22. Verwendung nach Anspruch 21, worin zusätzlich eine pharmazeutisch wirksame Menge wenigstens einer Verbindung verwendet wird, die ausgewählt ist unter Interleukin-2, $\beta$-Interferon, $\alpha$-Interferon und $\gamma$-Interferon.

23. Verwendung von Effektorzellen des peripheren Blutsystems, die zur Abtötung von HIV-infizierten Zellen befähigt sind, wobei die Effektorzellen mit wenigstens einem Antikörper-Heterokonjugat gemäß einem der Ansprüche 1 bis 10 in vitro behandelt wurden, und des Heterokonjugats zur Herstellung eines pharmazeutischen Mittels zur Behandlung von HIV-Infektionen.

24. Verwendung nach Anspruch 23, worin die Effektorzelle ausgewählt ist unter T-Lymphocyten, großen granulären Lymphocyten, Granulocyten, Monocyten und Makrophagen.

25. Verwendung nach Anspruch 23, worin die Effektorzellen vorbehandelt wurden mit einer Verbindung, die ausgewählt ist unter Interleukin-2, $\beta$-Interferon, $\alpha$-Interferon und $\gamma$-Interferon.

26. Verwendung nach Anspruch 23, worin die Effektorzellen mit einem Effektorzellen-spezifischen Antikörper vorbehandelt wurden.

27. Verwendung nach Anspruch 26, worin der Antikörper den lytischen Mechanismus der Effektorzelle stimuliert.

28. Verwendung nach Anspruch 27, worin der Antikörper ein Anti-CD3-Antikörper ist.

29. Verfahren zur Herstellung eines Antikörper-Heterokonjugats, umfassend wenigstens einen ersten Antikörper, der mit einem HIV-Antigen reagiert, das auf der Oberfläche einer HIV-infizierten Zelle exprimiert wird, der mit wenigstens einem zweiten Antikörper vernetzt ist, welcher mit einer Effektorzelle des peripheren Blutsystems reagiert, die zur Abtötung einer HIV-infizierten Zelle befähigt ist, wobei man den ersten und den zweiten Antikörper mit einem heterobifunktionellen Vernetzungsmittel umsetzt.

30. Verfahren nach Anspruch 29, wobei das Vernetzungsmittel N-Succinimidyl-3-(2-pyridylthio)-propionat (SPDP) oder Maleimidobutryloxysuccinimid (GMBS) ist.

31. Verfahren nach Anspruch 29, wobei der erste Antikörper 110.4 und der zweite Antikörper G19-4 ist; oder

der erste Antikörper 110.4 und der zweite Antikörper Fc2 ist; oder

der erste Antikörper 41.4 und der zweite Antikörper G19-4 ist.

**Revendications**
**Revendications pour les Etats contractants suivants : AT, BE, CH, DE, FR, GB, IT, LI, LU, NL, SE**

1. Hétéroconjugué d anticorps comprenant au moins un anticorps réactif avec un antigène VIH exprimé sur la surface d'une cellule infectée par le VIH réticulé à au moins un anticorps réactif avec une cellule d'effecteur du sang périphérique capable de tuer une cellule infectée par le VIH.

2. Hétéroconjugué d'anticorps selon la revendication 1, dans lequel l'antigène VIH est un de ceux trouvés sur une glycoprotéine enveloppe de VIH.

3. Hétéroconjugué d'anticorps selon la revendication 1, dans lequel la cellule d'effecteur est choisie parmi les lymphocytes T, les grands lymphocyte granulaires, les granulocytes, les monocytes et les macrophages.

4. Hétéroconjugué d'anticorps selon la revendication 1, dans lequel l'anticorps réactif avec une cellule d'effecteur est choisi parmi les anticorps réactifs avec le récepteur de cellule T sur dés lymphocytes T et un récepteur Fc sur des leucocytes.

5. Hétéroconjugué d'anticorps selon la revendication 1, dans lequel l'anticorps réactif avec la cellule d'effecteur est un anticorps à l'antigène CD3 sur le récepteur de cellule T de lymphocytes T.

6. Hétéroconjugué d'anticorps selon la revendication 1, dans lequel l'anticorps réactif avec une cellule d'effecteur est un anticorps pour récepteur Fc CD16 de grands lymphocytes granulaires et de granulocytes.

7. Hétéroconjugué d'anticorps selon la revendication 1, dans lequel les anticorps sont des fragments d'anticorps choisis parmi les fragments Fab et F(ab')$_2$.

8. Hétéroconjugué d'anticorps selon la revendication 1, dans lequel les anticorps sont des anticorps chimériques.

9. Hétéroconjugué d anticorps comprenant un premier anticorps réactif avec un antigène VIH exprimé sur la surface d'une cellule infectée par le VIH réticulé à un second anticorps réactif avec une cellule d'effecteur du sang périphérique capable de tuer une cellule infectée par le VIH.

10. Hétéroconjugué d'anticorps selon la revendication 9, choisi parmi le 110.4 x G19-4, 110.4 x Fc2 et 41.1 x G19-4.

11. Utilisation de cellules d'effecteur du sang périphérique en présence d'au moins un hétéroconjugué d'anticorps selon l'une des revendications 1 à 10 pour la préparation d'une composition pharmaceutique pour tuer les cellules infectées par le VIH.

12. Utilisation selon la revendication 11, dans laquelle les cellules d'effecteur sont choisies parmi les macrophages, monocytes, granulocytes et lymphocytes du sang périphérique.

**13.** Utilisation selon la revendication 11, dans laquelle les cellules d'effecteur sont obtenues à partir d'individus séropositifs ou séronégatifs VIH.

**14.** Utilisation selon la revendication 11, dans laquelle les cellules d'effecteur sont prétraitées avec un composé choisi parmi l'interleukine-2, le $\beta$-interféron, l'$\alpha$-interféron et le $\gamma$-interféron.

**15.** Utilisation selon la revendication 11, dans laquelle les cellules d'effecteur sont prétraitées avec un anticorps spécifique pour les cellules d'effecteur.

**16.** Utilisation selon la revendication 15, dans laquelle l'anticorps est un de ceux qui stimulent le mécanisme lytique des cellules d'effecteur.

**17.** Utilisation selon la revendication 16, dans laquelle l'anticorps est un anticorps anti-CD3.

**18.** Utilisation selon la revendication 11 ou 14, dans laquelle les cellules d'effecteur sont des lymphocytes T cytotoxiques et l'hétéroconjugué d'anticorps est choisi parmi le 110.4 x G19-4 et le 41.1 x G19-4.

**19.** Utilisation selon la revendication 11 ou 14, dans laquelle les cellules d'effecteur sont des grands lymphocytes granulaires et l'hétéroconjugué d'anticorps est le 110.4xFc2.

**20.** Composition pharmaceutiquement acceptable utile dans le traitement des infections VIH qui comprend une quantité efficace pharmaceutiquement d'au moins un hétéroconjugué d'anticorps selon l'une des revendications 1 à 10.

**21.** Utilisation d'une quantité pharmaceutiquement efficace d'au moins un hétéroconjugué d'anticorps selon l'une quelconque des revendications 1 à 10 pour la préparation d'une composition pharmaceutique pour traiter les infections VIH.

**22.** Utilisation selon la revendication 21, dans laquelle une quantité pharmaceutiquement efficace d'un composé choisi parmi l'interleukine-2, le $\beta$-interféron, l'$\alpha$-interféron et le $\gamma$-interféron est additionnellement employée.

**23.** Utilisation de cellules d'effecteur du sang périphérique capable de tuer des cellules infectées par le VIH, ces cellules d'effecteur ayant été traitées avec au moins un hétéroconjugué d'anticorps selon l'une des revendications 1 à 10 in vitro et dudit hétéroconjugué pour la préparation d'une composition pharmaceutique pour traiter les infections VIH.

**24.** Utilisation selon la revendication 23, dans laquelle la cellule d'effecteur est choisie parmi les lymphocytes T, les grands lymphocytes granulaires, les granulocytes, les monocytes et les macrophages.

**25.** Utilisation selon la revendication 23, dans laquelle les cellules d'effecteur sont prétraitées avec un composé choisi parmi l'interleukine-2, le $\beta$-interféron, l'$\alpha$-interféron et le $\gamma$-interféron.

**26.** Utilisation selon la revendication 23, dans laquelle les cellules d'effecteur sont prétraitées avec un anticorps spécifique pour les cellules d'effecteur.

**27.** Utilisation selon la revendication 26, dans laquelle l'anticorps est un de ceux qui stimulent le mécanisme lytique de la cellule d'effecteur.

**28.** Utilisation selon la revendication 27, dans laquelle l'anticorps est un anticorps anti-CD3.

**29.** Procédé pour préparer un hétéroconjugué d'anticorps ayant au moins un premier anticorps réactif avec un antigène VIH exprimé sur la surface d'un cellule infectée par le VIH réticulé à au moins un second anticorps réactif avec une cellule d'effecteur du sang périphérique capable de tuer une cellule infectée par le VIH, comprenant l'étape de réaction dudit premier et second anticorps avec un agent réticulant hétérobifonctionnel.

24

**30.** Procédé selon la revendication 29, dans lequel l'agent réticulant est le N-succinimidyl-3-(2-pyridylthio)-propionate (SPDP) ou le maléimidobutryloxysuccinimide (GMBS).

**31.** Procédé selon la revendication 29, dans lequel ledit premier anticorps est le 110.4 et ledit second anticorps est le G19-4;

ledit premier anticorps est le 110.4 et ledit second anticorps est le Fc2; ou

ledit premier anticorps est le 41.1 et ledit second anticorps est le G19-4.

**Revendications pour l'Etat contractant suivant : ES**

**1.** Procédé pour préparer un hétéroconjugué d'anticorps ayant au moins un premier anticorps réactif avec un antigène VIH exprimé sur la surface d'une cellule infectée par le VIH réticulé à au moins un second anticorps réactif avec une cellule d'effecteur du sang périphérique capable de tuer les cellules infectées par le VIH, comprenant l'étape de réaction dudit premier et second anticorps avec un agent réticulant hétérobifonctionnel.

**2.** Procédé selon la revendication 1, dans lequel l'antigène VIH est celui trouvé sur une glycoprotéine enveloppe de VIH.

**3.** Procédé selon la revendication 1, dans lequel la cellule d'effecteur est choisie parmi les lymphocytes T, les grands lymphocytes granulaires, les granulocytes, les monocytes et les macrophages.

**4.** Procédé selon la revendication 1, dans lequel l'anticorps réactif avec la cellule d'effecteur est choisi parmi les anticorps réactifs avec le récepteur de cellule T sur des lymphocytes T et un récepteur Fc sur des leucocytes.

**5.** Procédé selon la revendication 1, dans lequel l'anticorps réactif avec la cellule d'effecteur est un anticorps à l'antigène CD3 sur le récepteur de cellule T des lymphocytes T.

**6.** Procédé selon la revendication 1, dans lequel l'anticorps réactif avec la cellule d'effecteur est un anticorps au récepteur Fc CD16 des grands lymphocytes granulaires et des granulocytes.

**7.** Procédé selon la revendication 1, dans lequel les anticorps sont des fragments d'anticorps choisis parmi les fragments Fab et $F(ab')_2$.

**8.** Procédé selon la revendication 1, dans lequel les anticorps sont des anticorps chimériques.

**9.** Procédé selon l'une des revendications 1 à 8, dans lequel l'hétéroconjugué d'anticorps comprend un premier anticorps réactif avec un antigène VIH exprimé sur la surface d'une cellule infectée par le VIH réticulé à un second anticorps réactif avec une cellule d'effecteur du sang périphérique capable de tuer une cellule infectée par le VIH.

**10.** Procédé selon l'une des revendications 1 à 9, dans lequel ledit premier anticorps est le 110.4 et ledit second anticorps est le G19-4;

ledit premier anticorps est le 110.4 et ledit second anticorps est le Fc2; ou

ledit premier anticorps est le 41.1 et ledit second anticorps est le G19-4.

**11.** Procédé selon l'une des revendications 1 à 10, dans lequel l'agent réticulant est le N-succinimidyl-3-(2-pyrridylthio)-propionate (SPDP) ou le maléimidobutryloxysuccinimide (GMBS).

**12.** Utilisation de cellules d'effecteur du sang périphérique en présence d'au moins un hétéroconjugué d'anticorps préparé selon l'une des revendications 1 à 11 pour la préparation d'une composition pharmaceutique pour tuer les cellules infectées par le VIH.

**13.** Utilisation selon la revendication 12, dans laquelle les cellules d'effecteur sont choisies parmi les macrophages, les monocytes, les granulocytes et les lymphocytes du sang.

EP 0 308 936 B1

**14.** Utilisation selon la revendication 12, dans laquelle les cellules d'effecteur sont obtenues à partir d'individus séronégatifs ou séropositifs VIH.

**15.** Utilisation selon la revendication 12, dans laquelle les cellules d'effecteur sont prétraitées avec un composé choisi parmi l'interleukine-2, le $\beta$-interféron, l'$\alpha$-interféron et le $\gamma$-interféron.

**16.** Utilisation selon la revendication 12, dans laquelle les cellules d'effecteur sont prétraitées avec un anticorps spécifique pour les cellules d'effecteur.

**17.** Utilisation selon la revendication 16, dans laquelle l'anticorps est un de ceux qui stimulent le mécanisme lytique des cellules d'effecteur.

**18.** Utilisation selon la revendication 17, dans laquelle l'anticorps est un anticorps anti-CD3.

**19.** Utilisation selon la revendication 12 ou 15, dans laquelle les cellules d'effecteur sont des lymphocytes T cytotoxiques et l'hétéroconjugué d'anticorps est choisi parmi le 110.4 x G19-4 et le 41.1 x G19-4.

**20.** Utilisation selon la revendication 12 ou 15, dans laquelle les cellules d'effecteur sont des grands lymphocytes granulaires et l'hétéroconjugué d'anticorps est le 110.4 x Fc2.

**21.** Utilisation d'une quantité pharmaceutiquement efficace d'au moins un hétéroconjugué d'anticorps préparé selon l'une quelconque des revendications 1 à 11 pour la préparation d'une composition pharmaceutique pour le traitement des infections VIH.

**22.** Utilisation selon la revendication 21, dans laquelle une quantité pharmaceutiquement efficace d'un composé choisi parmi l'interleukine-2, le $\beta$-interféron, l'$\alpha$-interféron et le $\gamma$-interféron est additionnellement employée.

**23.** Utilisation des cellules d'effecteur du sang périphérique capables de tuer des cellules infectées par le VIH, lesquelles cellules d'effecteur ont été traitées avec au moins un hétéroconjugué d'anticorps préparé selon l'une quelconque des revendications 1 à 11 in vitro et dudit hétéroconjugué pour la préparation d'une composition pharmaceutique pour le traitement des infections VIH.

**24.** Utilisation selon la revendication 23, dans laquelle la cellule d'effecteur est choisie parmi les lymphocytes T, les grands lymphocytes granulaires, les granulocytes, les monocytes et les macrophages.

**25.** Utilisation selon la revendication 23, dans laquelle les cellules d'effecteur sont prétraitées avec un composé choisi parmi l'interleukine-2, le $\beta$-interféron, l'$\alpha$-interféron et le $\gamma$-interféron.

**26.** Utilisation selon la revendication 23, dans laquelle les cellules d'effecteur sont prétraitées avec un anticorps spécifique pour les cellules d'effecteur.

**27.** Utilisation selon la revendication 26, dans laquelle l'anticorps est un de ceux qui stimulent le mécanisme lytique de la cellule d'effecteur.

**28.** Utilisation selon la revendication 27, dans laquelle l'anticorps est un anticorps anti-CD3.

**29.** Procédé pour préparer une composition pharmaceutiquement acceptable utile dans le traitement d'une infection VIH qui comprend la fourniture d'une quantité pharmaceutiquement efficace d'au moins un hétéroconjugué d'anticorps préparé selon l'une des revendications 1 à 11.

**30.** Procédé selon la revendication 29, dans lequel une quantité efficace pharmaceutiquement d'un composé choisi parmi l'interleukine-2, le $\beta$-interféron, l'$\alpha$-interféron et le $\gamma$-interféron est additionnellement employée.

**31.** Procédé pour la préparation d'une composition pharmaceutique pour tuer les cellules infectées par le VIH qui comprend la fourniture de quantités efficaces pharmaceutiquement de cellules d'effecteur et d'hétéroconjugués d'anticorps tels que définis dans l'une des revendication 12 à 20.

26

**32.** Procédé pour préparer une composition pharmaceutique pour traiter les infections VIH, qui comprend la fourniture de quantités efficaces pharmaceutiquement de cellules d'effecteur et d'hétéroconjugués d'anticorps tels que définis dans l'une des revendications 23 à 28.

**Revendications pour l'Etat contractant suivant : GR**

**1.** Hétéroconjugué d'anticorps comprenant au moins un anticorps réactif avec un antigène VIH exprimé sur la surface d'une cellule infectée par le VIH réticulé à au moins un anticorps réactif avec une cellule d'effecteur du sang périphérique capable de tuer une cellule infectée par le VIH.

**2.** Hétéroconjugué d'anticorps selon la revendication 1, dans lequel l'antigène VIH est celui trouvé sur une glycoprotéine enveloppe de VIH.

**3.** Hétéroconjugué d'anticorps selon la revendication 1, dans lequel la cellule d'effecteur est choisie parmi les lymphocytes T, les grands lymphocytes granulaires, les granulocytes les monocytes et les macrophages.

**4.** Hétéroconjugué d'anticorps selon la revendication 1, dans lequel l'anticorps réactif avec la cellule d'effecteur est choisie parmi les anticorps réactifs avec le récepteur de cellule T sur les lymphocytes T ou un récepteur Fc sur les leucocytes.

**5.** Hétéroconjugué d'anticorps selon la revendication 1, dans lequel l'anticorps réactif avec la cellule d'effecteur est un anticorps à l'antigène CD3 sur le récepteur de cellule T des lymphocytes T.

**6.** Hétéroconjugué d'anticorps selon la revendication 1, dans lequel l'anticorps réactif avec la cellule d'effecteur est un anticorps au récepteur Fc CD16 de grands lymphocytes granulaires et de granulocytes.

**7.** Hétéroconjugué d'anticorps selon la revendication 1, dans lequel les anticorps sont des fragments d'anticorps choisis parmi les fragments Fab et F(ab')$_2$.

**8.** Hétéroconjugué d'anticorps selon la revendication 1, dans lequel les anticorps sont des anticorps chimériques.

**9.** Hétéroconjugué d'anticorps comprenant un premier anticorps réactif avec un antigène VIH exprimé sur la surface d'une cellule infectée par le VIH réticulé à un second anticorps réactif avec une cellule d'effecteur du sang périphérique capable de tuer une cellule infectée par le VIH.

**10.** Hétéroconjugué d'anticorps selon la revendication 9, choisi parmi le 110.4 x G19-4, le 110.4 x Fc2 et le 41,1 x G19-4.

**11.** Utilisation de cellules d'effecteur du sang périphérique en présence d'au moins un hétéroconjugué d'anticorps selon l'une des revendications 1 à 10 pour la préparation d'une composition pharmaceutique pour tuer les cellules infectées par le VIH.

**12.** Utilisation selon la revendication 11, dans laquelle les cellules d'effecteur sont choisies parmi les macrophages, monocytes, granulocytes et lymphocytes du sang périphérique.

**13.** Utilisation selon la revendication 11 dans laquelle les cellules d'effecteur sont obtenues à partir d'individus séropositifs ou séronégatifs VIH.

**14.** Utilisation selon la revendications 11, dans laquelle les cellules d'effecteur sont prétraitées avec un composé choisi parmi l'interleukine-2, le $\beta$-inteféron, l'$\alpha$-interféron et le $\gamma$-interféron.

**15.** Utilisation selon la revendication 11, dans laquelle les cellules d'effecteur sont prétraitées avec un anticorps spécifique pour les cellules d'effecteur.

**16.** Utilisation selon la revendication 15, dans laquelle l'anticorps est un de ceux qui stimulent le mécanisme lytique des cellules d'effecteur.

**17.** Utilisation selon la revendication 16, dans laquelle l'anticorps est un anticorps anti-CD3.

**18.** Utilisation selon la revendication 11 ou 14, dans laquelle les cellules d'effecteur sont des lymphocytes T cytotoxiques et l'hétéroconjugué d'anticorps est choisi parmi le 110.4 x G19-4 et le 41.1 x G19-4.

**19.** Utilisation selon la revendication 11 ou 14, dans laquelle les cellules d'effecteur sont des grands lymphocytes granulaires et l'hétéroconjugué d'anticorps est le 110.4 x Fc2.

**20.** Procédé pour préparer une composition pharmaceutiquement acceptable utile dans le traitement des infections VIH qui comprend la fourniture d'une quantité efficace pharmaceutiquement d'au moins un hétéroconjugué d'anticorps selon l'une des revendications 1 à 10.

**21.** Utilisation d'une quantité efficace pharmaceutiquement d'au moins un hétéroconjugué d'anticorps selon l'une quelconque des revendications 1 à 10 pour la préparation d'une composition pharmaceutique pour traiter les infections VIH.

**22.** Utilisation selon la revendication 21, dans laquelle une quantité efficace pharmaceutiquement d'un composé choisi parmi l'interleukine-2, le $\beta$-interféron, l'$\alpha$-interféron et le $\gamma$-interféron est additionnellement employée.

**23.** Utilisation de cellules d'effecteur du sang périphérique capable de tuer des cellules infectées par le VIH, lesquelles cellules d'effecteur ont été traitées avec au moins un hétéroconjugué d'anticorps selon l'une des revendications 1 à 10 in vitro et dudit hétéroconjugué pour la préparation d'une composition pharmaceutique pour traiter les infections VIH.

**24.** Utilisation selon la revendication 23, dans laquelle la cellule d'effecteur est choisie parmi les lymphocytes T, les grands lymphocytes granulaires, les granulocytes, les monocytes et les macrophages.

**25.** Utilisation selon la revendication 23, dans laquelle les cellules d'effecteur sont prétraitées avec un composé choisi parmi l'interleukine-2, le $\beta$-interféron, l'$\alpha$-interféron et le $\gamma$-interféron.

**26.** Utilisation selon la revendication 23, dans laquelle les cellules d'effecteur sont prétraitées avec un anticorps spécifique pour les cellules d'effecteur.

**27.** Utilisation selon la revendication 26, dans laquelle l'anticorps est un de ceux qui stimulent le mécanisme lytique de la cellule d'effecteur.

**28.** Utilisation selon la revendication 27, dans laquelle l'anticorps est un anticorps anti-CD3.

**29.** Procédé pour préparer un hétéroconjugué d'anticorps ayant au moins un premier anticorps réactif avec un antigène VIH exprimé sur la surface d'une cellule infectée par le VIH réticulé à au moins un second anticorps réactif avec une cellule d'effecteur du sang périphérique capable de tuer une cellule infectée par le VIH, comprenant l'étape de réaction dudit premier et second anticorps avec un agent réticulant hétérobifonctionnel.

**30.** Procédé selon la revendication 29, dans lequel l'agent réticulant est le N-succinimidyl-3-(2-pyridylthio)-propionate (SPDP) ou le maléimidobutryloxysuccinimide (GMBS).

**31.** Procédé selon la revendication 29, dans lequel ledit premier anticorps est le 110.4 et ledit second anticorps est le G19-4;

ledit premier anticorps est le 110.4 et ledit second anticorps est le Fc2; ou

ledit premier anticorps est le 41.1 et ledit second anticorps est le G19-4.

FIGURE 1

% Lysis of HIV Infected Cells

CD3-Activated PBL

— 110.4 x G19-4 conjugate
-- 110.4 + G19-4 mixture

Non-Activated PBL

Antibody Concentration
(ng/ml)

## FIGURE 2

A. Experi-

| ment Donor | % lysis of HIV-infected cells | | % lysis of uninfected cells | |
|---|---|---|---|---|
| | 110.4xG19-4 conjugate | 110.4+G19-4 mixture | 110.4xG19-4 conjugate | 110.4+G19-4 mixture |
| 1. A (sero-) | 42.9 | 14.5 | 3.5 | 9.5 |
| B (sero+) | 88.4 | 25.2 | 14.0 | 14.6 |
| 2. C (sero-) | 41.9 | 11.9 | 21.3 | 18.3 |
| D (sero-) | 27.7 | 8.8 | 15.1 | 13.6 |
| E (sero+) | 49.3 | 19.9 | 8.0 | 12.0 |
| F (sero+) | 22.5 | 4.4 | 2.7 | 8.7 |
| 3. G (sero-) | 23.5 | -2.4 | NT | NT |
| H (sero-) | 20.7 | 3.4 | NT | NT |

| B. CD3-activated Effector Cells | % lysis of HIV-infected cells | |
|---|---|---|
| E:T | 50:1 | 12.5:1 |
| Unseparated sero- cells | 24.3 | 6.1 |
| CD8[+] enriched cells | 36.0 | 24.6 |

FIGURE   3

## FIGURE 4

### % LYSIS OF HIV-INFECTED CELLS

| Antibody (200ng/ml) | Donor 366 PBL treated with IFN-β (U/ml) | | | Donor 372 PBL treated with IFN-β (U/ml) | | |
|---|---|---|---|---|---|---|
| | 1000 | 300 | 0 | 1000 | 300 | 0 |
| 110.4xFc2 conjugate | 41.3 | 26.9 | 18.7 | 37.6 | 44.0 | 27.4 |
| 110.4 alone | 9.7 | – | 7.1 | 23.4 | 16.4 | 12.3 |
| Fc2 alone | 2.1 | 7.3 | 2.4 | 18.6 | 7.9 | 9.7 |
| 110.4+Fc2 mixture | 11.6 | 7.3 | 3.1 | 18.0 | 11.1 | 12.7 |
| None | 10.2 | 5.1 | 2.4 | 15.0 | 8.0 | 5.8 |

# FIGURE    5

| Experi-ment | Donor | % lysis of HIV-infected cells | | % lysis of uninfected cells | |
|---|---|---|---|---|---|
| | | 110.4 x Fc2 conjugate | 110.4 + Fc2 mixture | 110.4 x Fc2 conjugate | 110.4 + Fc2 mixture |
| 1 | A (sero-) | 31.8 | 11.6 | 12.5 | 10.3 |
| | G (sero-) | 50.3 | 22.7 | 14.2 | 18.2 |
| | B (sero+) | 28.9 | 8.6 | 8.2 | 6.1 |
| 2 | C (sero-) | 60.2 | 28.4 | | |
| | D (sero-) | 35.5 | 18.3 | | |
| | E (sero+) | 38.0 | 17.5 | | |
| | F (sero+) | 30.1 | 8.6 | | |

FIGURE    6

FIGURE 7

## % LYSIS OF HIV-INFECTED CELLS

|                       | Donor 372 Antibody Concentration (ng/ml) | |
|-----------------------|------|------|
|                       | 200  | 50   |
| 41.1xG19-4 conjugate  | 23.0 | 15.4 |
| 41.1+G19-4 mixture    | 1.9  | 3.7  |
| 41.1 alone            | 3.9  | 3.3  |
| G19-4 alone           | -0.9 | 1.3  |